# EUROPÄISCHE PATENTSCHRIFT

(11) **EP 0 408 509 B1**
(45) Veröffentlichungstag und Bekanntmachung des Hinweises auf die Patenterteilung: **06.03.1996**
(21) Anmeldenummer: 90810515.8
(22) Anmeldetag: 05.07.1990
(51) Int. Cl.: C07D 257/04, C07D 403/06, A61K 31/41

(54) **Substituierte Benzonitrile**
Substituted benzonitriles
Benzonitriles substitués

(30) Priorität: 14.07.1989 CH 2644/89; 30.01.1990 CH 283/90
(43) Veröffentlichungstag der Anmeldung: 16.01.1991
(73) Patentinhaber: CIBA-GEIGY AG, CH-4002 Basel (CH)
(72) Erfinder: Lang, Marc, Dr., F-68170 Rixheim (FR)

(56) Entgegenhaltungen:
- EP-A- 0 165 778
- EP-A- 0 303 478
- EP-A- 0 348 257

## Beschreibung

Die Erfindung betrifft Verbindungen der Formel I, 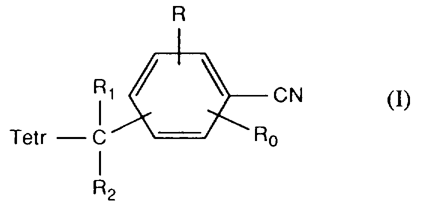 worin Tetr 1- oder 2-Tetrazolyl, welches unsubstituiert oder in 5-Stellung durch Niederalkyl, Phenylniederalkyl oder Niederalkanoyl substituiert ist, bedeutet; R₁ und R₂ unabhängig voneinander Wasserstoff; Niederalkyl, welches unsubstituiert oder durch Hydroxy, Niederalkoxy, Halogen, Carboxy, Niederalkoxycarbonyl, (Amino, Niederalkylamino oder Diniederalkylamino)-carbonyl oder Cyano substituiert ist; Niederalkenyl, Aryl, Hetaryl, Arylniederalkyl, C₃-C₆-Cycloalkyl, C₃-C₆-Cycloalkylniederalkyl, Niederalkylthio, Arylthio oder Arylniederalkylthio bedeuten; oder R₁ und R₂ zusammen C₄-C₆ geradkettiges Alkylen, welches unsubstituiert oder durch Niederalkyl substituiert ist, oder eine Gruppe -(CH₂)ₘ-1,2-Phenylen-(CH₂)ₙ-, worin m und n unabhängig voneinander 1 oder 2 bedeuten und 1,2-Phenylen unsubstituiert oder wie Phenyl gemäss der Definition von Aryl unten substituiert ist, oder Niederalkyliden, welches unsubstituiert oder durch Aryl mono- oder disubstituiert ist, bedeuten; und R und R₀ unabhängig voneinander Wasserstoff oder Niederalkyl bedeuten; oder R und R₀ zusammen, an benachbarten Kohlenstoffatomen des Benzolrings sitzend, eine Benzogruppe, die unsubstituiert oder wie Phenyl gemäss der Definition von Aryl unten substituiert ist, bedeuten; wobei in den obigen Definitionen Aryl jeweils Phenyl, welches unsubstituiert oder durch einen oder mehrere Substituenten aus der Gruppe bestehend aus Niederalkyl, Niederalkoxy, Hydroxy, Niederalkanoyloxy, Nitro, Amino, Halogen, Trifluormethyl, Carboxy, Niederalkoxycarbonyl, (Amino, Niederalkylamino oder Diniederalkylamino)-carbonyl, Cyano, Niederalkanoyl, Benzoyl, Niederalkylsulfonyl und (Amino, Niederalkylamino oder Diniederalkylamino)-sulfonyl substituiert ist, bedeutet; wobei in den obigen Definitionen Hetaryl einen aromatischen heterocyclischen Rest aus der Gruppe bestehend aus Pyrrolyl, Pyrazolyl, Imidazolyl, Triazolyl, Tetrazolyl, Furanyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl, Triazinyl, Indolyl, Isoindolyl, Benzimidazolyl, Benzotriazolyl, Benzofuranyl, Benzothienyl, Benzoxazolyl, Benzothiazolyl, Benzoxadiazolyl, Benzothiadiazolyl, Chinolyl und Isochinolyl, welcher unsubstituiert oder wie Phenyl gemäss der Definition von Aryl oben substituiert ist, bedeutet; und wobei in den oben genannten Definitionen "nieder" oder "Nieder" einen Rest bis und mit 7 Kohlenstoffatomen bedeutet; oder Salze davon, Verfahren zur Herstellung dieser Verbindungen, pharmazeutische Präparate, die diese Verbindungen enthalten, diese Verbindungen zur Anwendung zur prophylaktischen oder therapeutischen Behandlung des menschlichen oder tierischen Körpers oder ihre Verwendung zur Herstellung pharmazeutischer Präparate.

Die Verbindungen der Formel 1, welche ein asymmetrisches Kohlenstoffatom enthalten, können jeweils als Racemat oder als R- oder S-Enantiomer vorliegen. Die Erfindung bezieht sich auf alle diese Formen sowie z.B. auch auf Diastereomere und Gemische davon, die auftreten können, wenn zwei oder mehr asymmetrische Zentren im Molekül vorhanden sind, sowie auf geometrische Isomere, z.B. cis- und trans-Isomere, wenn das Molekül eine Doppelbindung enthält.

Die vor- und nachstehend verwendeten Allgemeinbegriffe haben im Rahmen der vorliegenden Anmeldung vorzugsweise die folgenden Bedeutungen:

Das Präfix "Nieder" bezeichnet einen Rest bis und mit 7, insbesondere bis und mit 4, und vor allen Dingen 1 oder 2 Kohlenstoffatomen.

Niederalkyl ist z.B. n-Propyl, Isopropyl, n-Butyl, Isobutyl, sek-Butyl, tert-Butyl, n-Pentyl, Neopentyl, n-Hexyl oder n-Heptyl, vorzugsweise Ethyl und vor allem Methyl.

Niederalkoxy ist z.B. Methoxy oder Ethoxy.

Halogen steht insbesondere für Chlor und Brom, kann aber auch Fluor oder Iod bedeuten.

Tetr bedeutet 1- oder 2-Tetrazolyl oder in 5-Stellung substituiertes 1- oder 2-Tetrazolyl. Insbesondere bedeutet Tetrazolyl 1- oder 2-Tetrazolyl.

Aryl ist Phenyl, das unsubstituiert oder durch einen oder mehrere, insbesondere einen oder zwei, Substituenten aus der Gruppe bestehend aus Niederalkyl, Niederalkoxy, Hydroxy, Niederalkanoyloxy, Nitro, Amino, Halogen, Trifluormethyl, Carboxy, Niederalkoxycarbonyl, (Amino, Niederalkylamino oder Diniederalkylamino)-carbonyl, Cyano, Niederalkanoyl, Benzoyl, Niederalkylsulfonyl und (Amino, Niederalkylamino oder Diniederalkylamino)-sulfonyl substituiert ist. In erster Linie bedeutet Aryl Phenyl, das unsubstituiert oder durch Niederalkyl, Niederalkoxy, Cyano oder Halogen substituiert ist, und vor allen Dingen Phenyl.

Arylniederalkyl ist z.B. Phenylniederalkyl und insbesondere Benzyl.

Hetaryl steht für einen wie unten definierten heterocyclischen aromatischen Rest, der üblicherweise über ein Kohlenstoffatom gebunden ist.

Hetarylreste mit 5 Ringatomen sind Pyrrolyl, Pyrazolyl, Imidazolyl, Triazolyl, Tetrazolyl, Furanyl, Thienyl, Isoxazolyl, Oxazolyl, Oxadiazolyl, Isothiazolyl, Thiazolyl oder Thiadiazolyl.

Hetarylreste mit 6 Ringatomen sind Pyridyl, Pyridazinyl, Pyrimidinyl, Pyrazinyl oder Triazinyl.

Hetarylreste bestehend aus einem heterocyclischen Ring und einem ankondensierten Benzoring sind Indolyl, Isoindolyl, Benzimidazolyl, Benzotriazolyl, Benzofuranyl, Benzothienyl, Benzoxazolyl, Benzothiazolyl, Benzoxadiazolyl, Benzothiadiazolyl, Chinolyl oder Isochinolyl.

Hetarylreste können unsubstituiert oder substituiert sein, z.B. so wie oben für Aryl angegeben. Hetarylreste können, z.B. abhängig von der Art der Substituenten, in verschiednen tautomeren Formen vorliegen.

Vorzugsweise bedeutet Hetaryl einen unsubstituierten oder wie oben für Aryl angegeben substituierten Rest aus der Gruppe bestehend aus Pyridyl, Thienyl, Indolyl und Furanyl oder einen Benzotriazolylrest der Formel (1), 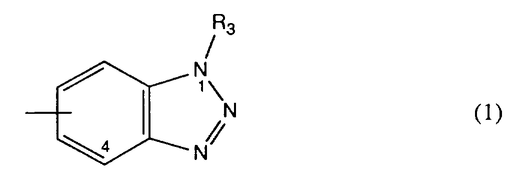 worin R₃ für Wasserstoff oder einen der oben für Aryl angegebenen Substituenten steht. Insbesondere bedeutet Hetaryl einen unsubstituierten oder durch Niederalkyl, Niederalkoxy, Cyano oder Halogen monosubstituierten Rest aus der Gruppe bestehend aus Pyridyl, Thienyl, Indolyl und Furanyl oder einen Benzotriazolylrest der Formel (1), worin R₃ für Wasserstoff, Niederalkyl, Hydroxy oder Niederalkoxy steht. In erster Linie bedeutet Hetaryl Pyridyl, Thienyl, Indolyl, Furanyl, oder in 1-Stellung unsubstituiertes oder durch Niederalkyl, Hydroxy oder Niederalkoxy substituiertes Benzotriazolyl, und vor allen Dingen 1-Niederalkyl-1H-benzotriazolyl.

Thienyl bedeutet z.B. 2- oder 3-Thienyl, und bevorzugt 2-Thienyl.

Pyridyl bedeutet z.B. 2-, 3- oder 4-Pyridyl, bevorzugt 3- oder 4-Pyridyl und insbesondere 3-Pyridyl.

Furanyl bedeutet z.B. 2- oder 3-Furanyl, und bevorzugt 3-Furanyl.

Indolyl bedeutet z.B. 3-Indolyl.

Benzotriazolyl bedeutet z.B. 4-, 5-, 6- oder 7-Benzotriazolyl, bevorzugt 5-, 6- oder 7-Benzotriazolyl und insbesondere 6-Benzotriazolyl.

Benzofuranyl bedeutet z.B. 4-, 5-, 6- oder 7-Benzofuranyl, bevorzugt 4-Benzofuranyl.

C₄-C₆ geradkettiges Alkylen gebildet aus den Gruppen R₁ und R₂ bedeutet vorzugsweise einen Rest -(CH₂)ₙ-, worin n für 4, 5 oder 6, insbesondere für 4 oder 5 steht, z.B. 1,4-Butylen oder vor allem 1,5-Pentylen, kann aber auch, z.B. durch Niederalkyl, substituiert sein.

Substituiertes Niederalkyl ist durch Hydroxy, Halogen, Niederalkoxy, Carboxy, Niederalkoxycarbonyl, (Amino, Niederalkylamino oder Diniederalkylamino)-carbonyl oder Cyano substituiert.

Niederalkanoyl(oxy) ist z.B. Formyl(oxy), Acetyl(oxy), Propionyl(oxy), n-Butyryl(oxy), Pivaloyl(oxy) oder Valeroyl(oxy).

Niederalkylsulfonyl ist z.B. Methylsulfonyl.

Bedeuten R und R₀ zusammen, an benachbarten Kohlenstoffatomen des Benzolrings sitzend, eine Benzogruppe, so bilden sie zusammen mit dem Benzolring ein Naphthalingerüst.

Arylniederalkylthio ist z.B. Phenylniederalkylthio und insbesondere Benzylthio.

Arylthio ist z.B. Phenylthio.

Niederalkylthio ist z.B. Methylthio oder Ethylthio.

Niederalkenyl ist z.B. Vinyl, Allyl, 1-Propenyl, Isopropenyl, 2- oder 3-Methallyl oder 3-Butenyl.

Niederalkyliden ist z.B. Methyliden oder Ethyliden.

C₃-C₆-Cycloalkylniederalkyl hat 3 bis 6 Ringkohlenstoffatome und ist z.B. Cyclopropylmethyl, Cyclopentylmethyl, Cyclohexylmethyl oder 2-Cyclohexylethyl.

Salze von erfindungsgemässen Verbindungen sind in erster Linie pharmazeutisch verwendbare, nicht-toxische Salze. Beispielsweise können Verbindungen der Formel I mit basischen Gruppen Säureadditionssalze bilden, z.B. mit anorganischen Säuren, wie Salzsäure, Schwefelsäure oder Phosphorsäure, oder mit geeigneten organischen Carbon- oder Sulfonsäuren, z.B. Essigsäure, Fumarsäure oder Methansulfonsäure, oder mit Aminosäuren, wie Arginin oder Lysin. Verbindungen der Formel I mit einer sauren Gruppe, z.B. 1-Tetrazolyl, bilden z.B. Metall- oder Ammoniumsalze, wie Alkalimetall- und Erdalkalimetall-, z.B. Natrium-, Kalium-, Magnesium- oder Calciumsalze, sowie Ammoniumsalze mit Ammoniak oder geeigneten organischen Aminen, wie Niederalkylaminen, z.B. Triethylamin, Hydroxyniederalkylaminen, z.B. 2-Hydroxyethylamin, Bis-(2-hydroxyethyl)-amin oder Tris-(2-hydroxyethyl)-amin, basischen aliphatischen Estern von Carbonsäuren, z.B. 4-Aminobenzoesäure-2-diethylaminoethylester, Niederalkylenaminen, z.B. 1-Ethylpiperidin, Cycloalkylaminen, z.B. Dicyclohexylamin, oder Benzylaminen, z.B. N,N′-Dibenzylethylendiamin, Dibenzylamin oder Benzyl-β-phenethylamin. Verbindungen der Formel I mit einer sauren und einer basischen Gruppe können auch in Form von inneren Salzen, d.h. in zwitterionischer Form, vorliegen.

Zur Isolierung oder Reinigung können auch pharmazeutisch ungeeignete Salze, z.B. Pikrate oder Perchlorate, Verwendung finden. Zur therapeutischen Anwendung gelangen nur die pharmazeutisch verwendbaren, nicht-toxischen Salze, die deshalb bevorzugt sind.

Die erfindungsgemässen Verbindungen der Formel I weisen wertvolle, insbesondere pharmakologisch verwendbare, Eigenschaften auf. Insbesondere hemmen sie selektiv das Enzym Aromatase bei Säugern einschliesslich Menschen. Dadurch wird die metabolische Umwandlung von Androgenen zu Oestrogenen gehemmt. Die Verbindungen der Formel I sind daher z.B. zur Behandlung Oestrogen-abhängiger Krankheiten, einschliesslich Oestrogen-abhängigem Brustkrebs, insbesondere bei postmenopausalen Frauen, geeignet. Ferner sind sie z.B. nützlich bei der Behandlung von Gynäkomastie, d.h. männlicher Brustentwicklung, weil die Aromatisierung der Steroide gehemmt wird.

Diese Wirkungen können durch in vitro- Versuche oder in vivo- Versuche, vorzugsweise an Säugetieren, z.B. Meerschweinchen, Mäusen, Ratten, Katzen, Hunden oder Affen, nachgewiesen werden. Die angewandte Dosierung liegt z.B. in einem Bereich von etwa 0,001 und 10 mg/kg, vorzugsweise zwischen 0,001 und 1 mg/kg.

Die in vitro-Hemmung der Aromataseaktivität kann z.B. mit der Methode, die in J. Biol. Chem. 249, 5364 (1974) beschrieben ist, gezeigt werden. Ferner können IC₅₀-Werte für die Aromatasehemmung z.B. in vitro aus enzymkinetischen Studien, die die Hemmung der Umwandlung von 4-¹⁴C-Androstendion zu 4-¹⁴C-Oestron in menschlichen plazentalen Mikrosomen betreffen, erhalten werden. Die IC₅₀-Werte der erfindungsgemässen Verbindungen liegen minimal etwa bei 10⁻⁹ M.

In vivo kann die Aromatasehemmung z.B. durch die Unterdrückung des ovarialen Oestrogengehalts weiblicher Ratten gezeigt werden, die zunächst mit Stutenserumgonadotropin und 2 Tage später mit menschlichem Choriongonadotropin injiziert werden, am folgenden Tag p.o. mit einer Verbindung der Erfindung behandelt werden und 1 h später mit Androstendion. Eine weitere Möglichkeit zur in vivo Bestimmung der Aromatasehemmung wird im folgenden beschrieben: Androstendion (30 mg/kg subkutan) wird allein bzw. zusammen mit einer Verbindung der Erfindung (oral oder subkutan) 4 Tage lang an nicht geschlechtsreife weibliche Ratten verabreicht. Nach der vierten Anwendung werden die Ratten getötet, die Uteri isoliert und gewogen. Die Aromatasehemmung wird bestimmt durch das Ausmass, in dem die durch die Verabreichung von Androstendion allein verursachte Uterushypertrophie durch die gleichzeitige Verabreichung der erfindungsgemässen Verbindung unterdrückt bzw. verringert wird. Die minimale effektive Dosis der erfindungsgemässen Verbindungen bei den in vivo Tests liegt etwa zwischen 0,001 und 1 mg/kg.

Die Antitumorwirkung, insbesondere bei Oestrogen-abhängigen Tumoren, kann in vivo z.B. bei DMBA-induzierten Mammatumoren an weiblichen Sprague-Dawley-Ratten gezeigt werden [vgl. Proc. Soc. Exp. Biol. Med. 160, 296-301 (1979)]. Die Anwendung von erfindungsgemässen Verbindungen bewirkt eine Regression der Tumoren und unterdrückt weiterhin das Auftreten neuer Tumoren bei täglichen Dosen ab etwa 1 mg/kg p.o.

Darüberhinaus haben die Verbindungen der Formel I keine Hemmwirkung auf die Spaltung der Cholesterin-Seitenkette und induzieren keine adrenale Hypertrophie, was durch endokrine Organuntersuchungen gezeigt wird.

Aufgrund ihrer pharmakologischen Eigenschaften als äusserst selektive Inhibitoren des Enzyms Aromatase sind die Verbindungen der Formel I z.B. zur Behandlung Oestrogen-abhängiger Krankheiten, wie Brusttumoren (Brustkarzinoma), Endometriosis, vorzeitigen Wehen oder endometrialen Tumoren bei Frauen oder Gynäkomastie bei Männern, geeignet.

Besonders bevorzugt sind die Verbindungen der Formel I, worin Tetr 1- oder 2-Tetrazolyl bedeutet; R₁ und R₂ unabhängig voneinander Wasserstoff; Niederalkyl, welches unsubstituiert oder durch Hydroxy, Niederalkoxy, Halogen, Carboxy, Niederalkoxycarbonyl, (Amino, Niederalkylamino oder Diniederalkylamino)-carbonyl oder Cyano substituiert ist; Niederalkenyl, Aryl, Hetaryl, Arylniederalkyl, C₃-C₆-Cycloalkyl, C₃-C₆-Cycloalkylniederalkyl, Niederalkylthio, Arylthio oder Arylniederalkylthio bedeuten; oder R₁ und R₂ zusammen C₄-C₆ geradkettiges Alkylen, welches unsubstituiert oder durch Niederalkyl substituiert ist, oder eine Gruppe -(CH₂)ₘ-1,2-Phenylen-(CH₂)ₙ-, worin m und n unabhängig voneinander 1 oder 2 bedeuten und 1,2-Phenylen unsubstituiert oder wie Phenyl gemäss der Definition von Aryl unten substituiert ist, oder Niederalkyliden, welches unsubstituiert oder durch Aryl mono- oder disubstituiert ist, bedeuten; und R und R₀ unabhängig voneinander Wasserstoff oder Niederalkyl bedeuten; oder R und R₀ zusammen, an benachbarten Kohlenstoffatomen des Benzolrings sitzend, eine Benzogruppe, die unsubstituiert oder wie Phenyl gemäss der Definition von Aryl unten substituiert ist, bedeuten; wobei in den obigen Definitionen Aryl jeweils Phenyl, welches unsubstituiert oder durch einen oder zwei Substituenten aus der Gruppe bestehend aus Niederalkyl, Niederalkoxy, Hydroxy, Niederalkanoyloxy, Nitro, Amino, Halogen, Trifluormethyl, Carboxy, Niederalkoxycarbonyl, (Amino, Niederalkylamino oder Diniederalkylamino)-carbonyl, Cyano, Niederalkanoyl, Benzoyl, Niederalkylsulfonyl und (Amino, Niederalkylamino oder Diniederalkylamino)-sulfonyl substituiert ist, bedeutet; wobei in den obigen Definitionen Hetaryl einen aromatischen heterocyclischen Rest aus der Gruppe bestehend aus Thienyl, Indolyl, Pyridyl, Furyl und Benzofuranyl, welcher unsubstituiert oder durch 1 bis 3 Substituenten aus der Gruppe bestehend aus Niederalkyl, Niederalkoxy, Cyano und Halogen substituiert ist, oder in 1-Stellung unsubstituiertes oder durch Niederalkyl, Hydroxy oder Niederalkoxy substituiertes Benzotriazolyl bedeutet; und Salze davon.

In erster Linie bevorzugt sind die Verbindungen der Formel I, worin Tetr 1- oder 2-Tetrazolyl bedeutet; R₁ und R₂ unabhängig voneinander Wasserstoff; Niederalkyl, welches unsubstituiert oder durch Niederalkoxycarbonyl substituiert ist; Phenyl, welches unsubstituiert oder durch Cyano, Halogen, Niederalkoxy, Niederalkyl oder Hydroxyniederalkyl substituiert ist; Thienyl, Pyridyl; 1-Niederalkyl-1H-benzotriazolyl; Phenylniederalkyl, welches im Phenylring unsubstituiert oder durch Cyano substituiert ist; Niederalkylthio oder Phenylthio bedeuten; oder R₁ und R₂ zusammen C₄-C₅ geradkettiges Alkylen oder eine Gruppe -CH₂-1,2-Phenylen-CH₂- bedeuten; und R und R₀ Wasserstoff bedeuten; oder R und R₀ zusammen, an benachbarten Kohlenstoffatomen des Benzolrings sitzend, eine Benzogruppe bedeuten; und Salze davon.

Vor allen Dingen bevorzugt sind die Verbindungen der Formel I, worin Tetr 1- oder 2-Tetrazolyl bedeutet, R₁ Wasserstoff, Niederalkyl; Phenyl, welches unsubstituiert oder durch Cyano, Halogen, Niederalkoxy oder Niederalkyl substituiert ist; oder Phenylniederalkyl bedeutet; und R, R₀ und R₂ für Wasserstoff stehen, und Salze davon.

Ferner bevorzugt sind die Verbindungen der Formel I, worin Tetr 1- oder 2-Tetrazolyl bedeutet; R₁ Wasserstoff; Niederalkyl, welches unsubstituiert oder durch Hydroxy, Niederalkoxy, Halogen, Carboxy, Niederalkoxycarbonyl, (Amino, Niederalkylamino oder Diniederalkylamino)-carbonyl oder Cyano substituiert ist; Niederalkenyl, Aryl, Hetaryl, Arylniederalkyl, C₃-C₆-Cycloalkyl, C₃-C₆-Cycloalkylniederalkyl, Niederalkylthio, Arylthio oder Arylniederalkylthio bedeutet; R₂ für Wasserstoff steht; oder R₁ und R₂ zusammen C₄-C₆ geradkettiges Alkylen, welches unsubstituiert oder durch Niederalkyl substituiert ist, oder eine Gruppe -(CH₂)ₘ-1,2-Phenylen-(CH₂)ₙ-, worin m und n unabhängig voneinander 1 oder 2 bedeuten und 1,2-Phenylen unsubstituiert oder wie Phenyl gemäss der Definition von Aryl unten substituiert ist, oder Niederalkyliden, welches unsubstituiert oder durch Aryl mono- oder disubstituiert ist, bedeuten; und R und R₀ unabhängig voneinander Wasserstoff oder Niederalkyl bedeuten; oder R und R₀ zusammen, an benachbarten Kohlenstoffatomen des Benzolrings sitzend, eine Benzogruppe, die unsubstituiert oder wie Phenyl gemäss der Definition von Aryl unten substituiert ist, bedeuten; wobei in den obigen Definitionen Aryl jeweils Phenyl, welches unsubstituiert oder durch einen Substituenten aus der Gruppe bestehend aus Niederalkyl, Niederalkoxy, Hydroxy, Niederalkanoyloxy, Nitro, Amino, Halogen, Trifluormethyl, Carboxy, Niederalkoxycarbonyl, (Amino, Niederalkylamino oder Diniederalkylamino)-carbonyl, Cyano, Niederalkanoyl, Benzoyl, Niederalkylsulfonyl und (Amino, Niederalkylamino oder Diniederalkylamino)-sulfonyl substituiert ist, bedeutet; wobei in den obigen Definitionen Hetaryl einen aromatischen heterocyclischen Rest aus der Gruppe bestehend aus Thienyl, Indolyl, Pyridyl, Furyl und Benzofuranyl, welcher unsubstituiert oder durch 1 oder 2 Substituenten aus der Gruppe bestehend aus Niederalkyl, Niederalkoxy, Cyano und Halogen substituiert ist, oder in 1-Stellung unsubstituiertes oder durch Niederalkyl, Hydroxy oder Niederalkoxy substituiertes Benzotriazolyl bedeutet; und Salze davon.

Insbesondere bevorzugt sind die Verbindungen der Formel I, worin Tetr 1- oder 2-Tetrazolyl bedeutet; R₁ Wasserstoff; Niederalkyl, welches unsubstituiert oder durch Niederalkoxycarbonyl substituiert ist; Phenyl, welches unsubstituiert oder durch Cyano, Halogen, Niederalkoxy, Niederalkyl oder Hydroxyniederalkyl substituiert ist; Thienyl, Pyridyl; Phenylniederalkyl, welches im Phenylring unsubstituiert oder durch Cyano substituiert ist; Niederalkylthio oder Phenylthio bedeutet; R₂ für Wasserstoff steht; oder R₁ und R₂ zusammen C₄-C₅ geradkettiges Alkylen oder eine Gruppe -CH₂-1,2-Phenylen-CH₂-bedeuten; und R und R₀ Wasserstoff bedeuten; oder R und R₀ zusammen, an benachbarten Kohlenstoffatomen des Benzolrings sitzend, eine Benzogruppe bedeuten; und Salze davon.

Als Untergruppen aus einer Gruppe von Verbindungen der Formel I sind jeweils hervorzuheben: (a) Verbindungen der Formel I, worin der Rest Tetr 2-Tetrazolyl bedeutet; (b) Verbindungen der Formel I, worin der Rest Tetr 1-Tetrazolyl bedeutet; (c) Verbindungen der Formel I, worin der Rest -CR₁R₂(Tetr) in p-Stellung zur Cyanogruppe angeknüpft ist; (d) Verbindungen der Formel I, worin R₂ Wasserstoff bedeutet; (e) Verbindungen der Formel I, worin R₁ Phenyl, welches unsubstituiert oder durch Cyano, Halogen, Niederalkoxy oder Niederalkyl monosubstituiert ist, bedeutet und R₂ für Wasserstoff steht; (f) Verbindungen der Formel I, worin R und R₀ Wasserstoff bedeuten.

Die Erfindung betrifft vor allem die in den Beispielen beschriebenen spezifischen Verbindungen und pharmazeutisch verwendbare Salze davon.

Die Verbindungen der Formel I können in an sich bekannter Weise hergestellt werden, indem man z.B.
(a) ein reaktives verestertes Derivat einer Verbindung der Formel II, 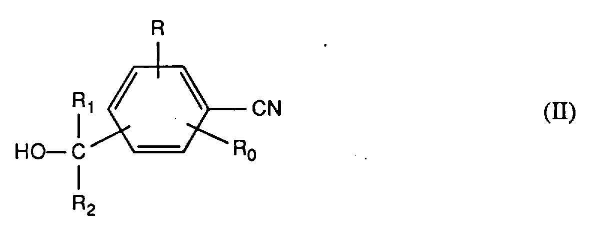 worin R, R₀, R₁ und R₂ wie unter Formel I definiert sind, mit einer Verbindung der Formel III,

   Tetr-H (III)

   worin Tetr Tetrazolyl bedeutet, oder einem N-geschützten Derivat davon, umsetzt, oder
(b) zur Herstellung von Verbindungen der Formel I, worin Tetr 1-Tetrazolyl bedeutet, in einer Verbindung der Formel IV, 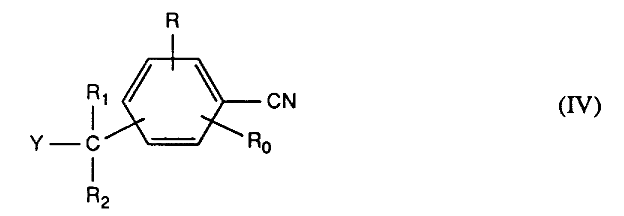 worin Y einen in 1-Tetrazolyl überführbaren Rest bedeutet und R, R₀, R₁ und R₂ wie unter Formel I definiert sind, Y in 1-Tetrazolyl umwandelt, oder
(c) eine Verbindung der Formel V, 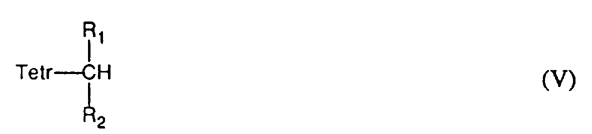 worin Tetr, R₁ und R₂ wie unter Formel I definiert sind, im basischen Medium mit einer Verbindung der Formel VI, 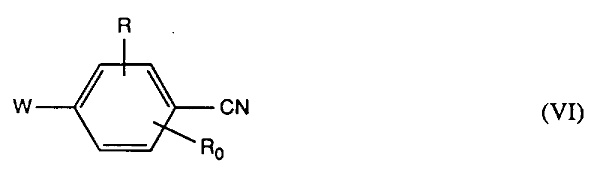 worin W eine Abgangsgruppe bedeutet und R und R₀ wie unter Formel I definiert sind, umsetzt, oder
(d) in einer Verbindung der Formel VII, 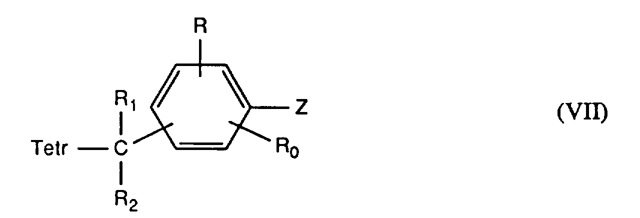 worin Z einen in Cyano überführbaren Rest bedeutet und Tetr, R, R₀, R₁ und R₂ wie unter Formel I definiert sind, den Rest Z in eine Cyanogruppe überführt; und/oder, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I in ein Salz umwandelt, und/oder ein erhaltenes Gemisch von isomeren Verbindungen der Formel I in die einzelnen Isomeren auftrennt.

In der folgenden näheren Beschreibung der Verfahren (a), (b), (c) und (d) haben die Symbole Tetr, R, R₀, R₁ und R₂ jeweils die unter Formel I angegebene Bedeutung, sofern nichts anderes angegeben ist.

Verfahren (a): In einer Verbindung der Formel II bedeutet ein reaktives verestertes Derivat der Hydroxymethyl-Gruppe -CR₁R₂OH (gegebenenfalls substituiertes) Hydroxymethyl, das durch eine Abgangsgruppe verestert ist, z.B. Niederalkyl- oder Aryl-sulfonyloxymethyl, wie Methylsulfonyloxymethyl oder p-Toluolsulfonyloxymethyl, oder Halogenmethyl, z.B. Chlormethyl, Brommethyl oder Iodmethyl.

Setzt man bei der Umsetzung gemäss Verfahren (a) Tetrazol als Verbindung der Formel III ein, so erhält man normalerweise Gemische von Verbindungen der Formel I, worin Tetr 1-Tetrazolyl, 2-Tetrazolyl bzw. 5-Tetrazolyl bedeutet, die sich leicht z.B. durch Chromatographie auftrennen lassen. In einigen Fällen ist es möglich, durch die Verwendung von Verbindungen der Formel III, worin ein bestimmtes Ringstickstoffatom durch eine Schutzgruppe geschützt ist, selektiv nur eine der jeweils in Frage kommenden Verbindungen zu erhalten.

Geeignete Schutzgruppen für ein Ringstickstoffatom in einer Verbindung der Formel III sind z.B. Triniederalkylsilyl, z.B. Trimethylsilyl, Niederalkanoyl, z.B. Acetyl, Diniederalkylaminocarbonyl, z.B. Dimethylaminocarbonyl, oder Triarylmethyl, z.B. Triphenylmethyl.

Die Kondensationsreaktion gemäss Verfahren (a) ist an sich bekannt und entspricht einer gewöhnlichen N-Alkylierung, die z.B. ohne Zusatz von Basen oder vorzugsweise in Gegenwart von Basen, wie z.B. Kaliumcarbonat, Natrium, Triethylamin oder Pyridin, durchgeführt wird.

Reaktive veresterte Derivate von Verbindungen der Formel II sind an sich bekannt oder werden z.B. in an sich bekannter Weise aus den entsprechenden Hydroxymethyl-Verbindungen durch Veresterung erhalten. Die Hydroxymethyl-Verbindungen sind z.B. durch Reduktion, z.B. mit LiAlH₄ oder Diisobutylaluminiumhydrid, aus den entsprechenden Carboxy- oder Niederalkoxycarbonylverbindungen erhältlich. Letztere sind an sich bekannt oder können analog zu bekannten substituierten Cyano-benzoesäuren bzw. -estern hergestellt werden.

Die Umsetzung gemäss Verfahren (a) dient insbesondere zur Herstellung von Verbindungen der Formel I, worin R₁ und R₂ Wasserstoff bedeuten.

Verfahren (b): Ein in 1-Tetrazolyl überführbarer Rest Y ist z.B. Isocyano (-N^{⊕}≡C^{⊖}) oder Amino.

Verbindungen der Formel IV, worin Y Isocyano bedeutet, (Isonitrile) werden z.B. durch Umsetzung mit Stickstoffwasserstoffsäure oder insbesondere einem Salz davon, z.B. einem Alkalimetall- oder Ammoniumazid, in die entsprechenden 1-Tetrazolylverbindungen der Formel I überführt.

Verbindungen der Formel IV, worin Y Amino bedeutet, werden z.B. durch Umsetzung mit Stickstoffwassersäure, oder insbesondere einem Salz davon, und einem Orthoameisensäuretriniederalkylester, z.B. Orthoameisensäuretriethylester, in die entsprechenden 1-Tetrazolylverbindungen der Formel I überführt.

Isonitrile der Formel IV werden z.B. aus den analogen reaktiven veresterten Derivaten von Verbindungen der Formel II, worin die veresterte -CR₁R₂OH-Gruppe z.B. gegebenenfalls α,α′-substituiertes Brommethyl bedeutet, hergestellt. Letztere werden in an sich bekannter Weise z.B. direkt mit Silbercyanid in einem polaren Lösungsmittel oder zunächst, z.B. durch Umsetzung mit Hexamethylentetramin (Urotropin), in die entsprechenden gegebenenfalls α,α'-substituierten Aminomethyl-Verbindungen (= Verbindungen der Formel IV, worin Y = Amino) und dann in an sich bekannter Weise, z.B. durch Umsetzung mit Dichlorcarben (z.B. aus Chloroform und konz. KOH), in die gewünschten Isonitrile der Formel IV überführt

Verfahren (c): Eine Abgangsgruppe W in einer Verbindung der Formel VI ist z.B. Halogen, Niederalkyl- oder Arylsulfonyloxy und bevorzugt Fluor.

Als Basen kommen bei Ihrer Umsetzung gemäss Verfahren (c) vor allem starke Basen in Frage, z.B. Lithiumdiisopropylamid, ein Alkalimetallhydrid, ein Alkalimetallniederalkoxid, z.B. Kalium-tert-butoxid (= Kalium-tert-butylat), oder ein stark basisches tertiäres Amin, z.B. 1,5-Diazabicyclo[4.3.0]non-5-en (DBN).

Die Ausgangsverbindungen der Formel V werden z.B. durch Umsetzung einer Verbindung der Formel VIII 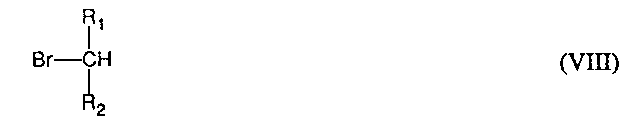 mit Tetrazol erhalten.

Verfahren (d): Das Verfahren (d) wird gemäss bekannten Methoden zur Einführung der Nitrilgruppe durchgeführt.

Zu den Resten Z in einer Verbindung der Formel VII, die in Cyano überführt werden können, gehören z.B. Wasserstoff; veresterte Hydroxygruppen, z.B. Halogen, insbesondere Chlor, Brom oder Iod, oder eine Sulfonyloxygruppe, z.B. Toluolsulfonyloxy, Benzolsulfonyloxy oder Methylsulfonyloxy; Sulfo (-SO₃H), Amino, Carboxy, Carboxy in Form eines funktionellen Derivates, z.B. Aminocarbonyl, Niederalkylaminocarbonyl, z.B. tert-Butylaminocarbonyl, oder Halogenformyl, z.B. Chlor- oder Bromformyl (-COCl, -COBr), Formyl in Form eines funktionellen Derivates, z.B. Hydroxyiminomethyl, oder Halogenmagnesium, z.B. Iod-, Brom- oder Chlormagnesium.

Verbindungen der Formel I können gemäss Verfahren (d) z.B. durch die folgenden Umsetzungen erhalten werden:

Die Umsetzung einer Verbindung der Formel VII, worin Z Wasserstoff bedeutet, in das entsprechende Nitril der Formel I erfolgt z.B. nach der bekannten Methode von C. Friedel, F.M. Crafts und P. Karrer mit Chlorcyan (ClCN) oder Bromcyan, oder nach der Methode von J. Houben und W. Fischer z.B. mit Trichloracetonitril. Vorteilhafterweise wird hierbei der übliche Katalysator, Aluminiumtrichlorid, verwendet. Bei diesen Reaktionen wird Chlorwasserstoff oder Bromwasserstoff freigesetzt, der aus dem Reaktionsgemisch durch Zugabe einer Base, vorzugsweise eines Amins, z.B. Triethylamin oder Pyridin, entfernt werden kann.

Die Umsetzung einer Verbindung der Formel VII, worin Z Halogen bedeutet, z.B. Chlor, Brom oder Iod, in ein entsprechendes Nitril der Formel I wird z.B. unter Verwendung eines Cyanidsalzes, insbesondere von Natrium- oder Kaliumcyanid oder vorzugsweise Kupfer(I)cyanid, durchgeführt. Bevorzugte Lösungsmittel für diese Reaktion sind Pyridin, Chinolin, Dimethylformamid, 1-Methyl-2-pyrrolidon und Hexamethylphosphorsäuretriamid. Bevorzugt werden hohe Temperaturen, insbesondere die Rückflusstemperatur des Reaktionsgemisches.

Die Umsetzung einer Verbindung der Formel VII, worin Z Sulfonyloxy bedeutet, z.B. p-Toluolsulfonyloxy, Benzolsulfonyloxy oder Methylsulfonyloxy, in ein Nitril der Formel I wird z.B. durch Reaktion mit einem Alkalimetallcyanid, vorzugsweise Natrium - oder Kaliumcyanid, durchgeführt. Bevorzugt werden dabei hohe Temperaturen, insbesondere die Rückflusstemperatur des Reaktionsgemisches.

Die Umsetzung einer Verbindung der Formel VII, worin Z Amino bedeutet, in ein Nitril der Formel I verläuft über mehrere Stufen. Zuerst wird z.B. ein Diazoniumsalz hergestellt, z.B. durch Umsetzung der Aminoverbindung mit einem Alaklimetallnitrit, vorzugsweise Kaliumnitrit. Das Diazoniumsalz kann dann unter Verwendung der bekannten Sandmeyer-Reaktion in situ weiter umgesetzt werden, z.B. mit Kupfer(I)cyanid oder Einem komplexen Cyanid, vorzugsweise Kalium-kupferammoniumcyanid, oder mit einer katalytischen Menge frisch gefällten Kupferpulvers in Gegenwart eines Alkalimetallcyanids, z.B. Natrium- oder Kaliumcyanid.

Die Umsetzung einer Verbindung der Formel VII, worin Z Carboxy bedeutet, in ein Nitril der Formel I kann z.B. durch Reaktion mit einem Chlorsulfonylisocyanat in z.B. Dimethylformamid nach der Methode von R. Graf in Angew. Chem. 80, 183 (1968) durchgeführt werden.

Die Umsetzung einer Verbindung der Formel VII, worin Z Carboxy bedeutet, das in Form eines funktionellen Derivates vorliegt, z.B. als Aminocarbonyl oder Niederalkylaminocarbonyl, vorteilhafterweise tert-Butylaminocarbonyl, in ein Nitril der Formel I kann z.B. mit einem starken Dehydratisierungsmittel, z.B. Phosphor(V)oxid, Phosphorylchlorid, Thionylchlorid, Phosgen odere Oxalylchlorid, durchgeführt werden. Die Dehydratisierung kann vorzugsweise in Gegenwart einer Base durchgeführt werden. Eine geeignete Base ist z.B. ein Amin, z.B. ein tertiäres Amin, etwa ein Triniederalkylamin wie Trimethylamin, Triethylamin oder Ethyldiisopropylamin, oder ein Arylalkylamin, z.B. N,N-Dimethylanilin, oder ein zyklisches tertiäres Amin, z.B. ein Niederalkyl-morpholin, z.B. N-Methylmorpholin, oder z.B. eine Base vom Typ des Pyridins, z.B. Pyridin oder Chinolin.

Die Umsetzung einer Verbindung der Formel VII, worin Z Formyl bedeutet, in ein nitril der Formel I wird z.B. durch Umsetzung der Formylgruppe in ein reaktives, funktionelles Derivat, z.B. Hydroxyiminomethyl, und Umwandlung dieser Gruppe durch ein Dehydratisierungsmittel in eine Cyangruppe durchgeführt. Ein geeignetes Dehydratisierungsmittel ist eines der oben genannten anorganischen Dehydratisierungsmittel, z.B. Phosphor(V)-chlorid, oder vorzugsweise das Anhydrid einer organischen Säure, z.B. das Anhydrid einer Niederalkansäure, z.B. Essigsäureanhydrid. Die Umsetzung der Formylgruppe in die hydroxyiminomethylgruppe erfolgt z.B. durch Reaktion mit einem Salz des Hydroxylamins, vorzugsweise dem Hydrochlorid.

Eine Verbindung der Formel VII, worin Z Formyl bedeutet, kann auch direkt in das entsprechende Nitril der Formel I umgesetzt werden, z.B. durch Reaktion mit O,N-Bis-(trifluoracetyl)-hydroxylamin in Gegenwart einer Base, z.B. Pyridin, nach der Methode von D.T. Mowry, Chem. Revs. 42, 251 (1948).

Die Umsetzung einer Verbindung der Formel VII, worin Z Halogenmagnesium bedeutet, z.B. Iod-, Brom- oder Chlormagnesium, in ein entsprechendes Nitril der Formel I wird z.B. so ausgeführt, dass ein Magnesiumhalogenid mit Halogencyan oder Dicyan zur Reaktion gebracht wird. Das "Grignard"-Reagens, also eine Verbindung der Formel VII, worin Z eine Halogenmagnesiumgruppe ist, wird nach üblichen Verfahren hergestellt, z.B. durch Umsetzung einer Verbindung der Formel VII, worin Z Halogen, z.B. Chlor, Brom oder Iod bedeutet, mit Magnesium, z.B. in trockenem Ether.

Verbindungen der Formel VII, worin Tetr 1- oder 2-Tetrazolyl bedeutet, R₁ einen Benzotriazolylrest der Formel (1), 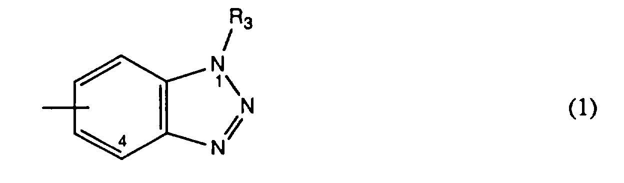 worin R₃ wie oben unter Formel (1) definiert ist, bedeutet, R, R₀ und R₂ Wasserstoff bedeuten, oder R und R₀ zusammen, an benachbarten Kohlenstoffatomen des Benzolrings sitzend, eine Benzogruppe bedeuten und Z für Chlor oder Brom steht, und Salze davon, sind wertvolle Zwischenprodukte zur Herstellung der entsprechenden Cyanoverbindungen der Formel I. Darüberhinaus sind sie ebenso wie die Verbindungen der Formel I als Aromatasehemmer wirksam. Sie bilden einen weiteren Gegenstand der Erfindung.

Verbindungen der Formel I können in andere Verbindungen der Formel I umgewandelt werden.

Zum Beispiel können Verbindungen der Formel I, worin R₁ und R₂ Wasserstoff bedeuten, durch Umsetzung mit einem reaktiven funktionellen Derivat von R₁ oder R₂ (R₁ ≠ H, R₂ ≠ H) in Verbindungen der Formel I, worin einer der Reste R₁ und R₂ Wasserstoff bedeutet und der andere der Reste R₁ und R₂ von Wasserstoff verschieden ist, überführt werden.

Verbindungen der Formel I, worin einer der Reste R₁ und R₂ Wasserstoff bedeutet und der andere der Reste R₁ und R₂ von Wasserstoff verschieden ist, können ihrerseits durch Umsetzung mit einem reaktiven funktionellen Derivat von R₁ oder R₂ (R₁ ≠ H, R₂ ≠ H) in Verbindungen der Formel I, worin R₁ und R₂ von Wasserstoff verschieden sind, überführt werden.

Ferner können Verbindungen der Formel I, worin R₁ und R₂ Wasserstoff bedeuten, durch Umsetzung mit einem reaktiven funtionellen Derivat von R₁ oder R₂ (R₁ ≠ H, R₂ ≠ H) bzw. einem bifunktionellen Derivat eines aus R₁ und R₂ gemeinsam gebildeten Restes, in Verbindungen der Formel I, worin R₁ und R₂ von Wasserstoff verschieden sind und worin entweder R₁ mit R₂ identisch ist oder R₁ und R₂ gemeinsam einen Rest wie unter Formel I definiert bilden, überführt werden.

Funktionelle Derivate von R₁ und/oder R₂ sind z.B. Halogen-, etwa Chlor- oder Brom- oder Sulfonyloxyderivate, etwa Niederalkylsulfonyloxy oder Arylsulfonyloxy.

Die entsprechenden Kondensationsreaktionen werden in an sich bekannter Weise z.B. so ausgeführt, dass zuerst in Gegenwart einer starken Base, z.B. Lithiumdiisopropylamid, einem Alkalimetallhydrid, einem Alkalimetallniederalkoxid wie Kalium-tert-butoxid, oder einem stark basischen tertiären Amin wie 1,5-Diazabicyclo[4.3.0]non-5-en (DBN), vorzugsweise unter einer Schutzgasatmosphäre, z.B. unter einer Stickstoffatmosphäre, und in einem inerten Lösungsmittel, z.B. in Dimethylformamid, ein Carbanion der Verbindung der Formel I mit R₁ = R₂ = H bzw. R₁ = H, R₂ ≠ H oder R₁ ≠ H, R₂ = H hergestellt wird. Letzteres wird dann mit einem funktionellen Derivat von R₁ oder R₂ bzw. R₁ + R₂ umgesetzt.

Für Verbindungen der Formel I, in denen R₁ und/oder R₂ 4-Cyanophenyl darstellt, ist ein geeignetes reaktives Derivat p-Fluorbenzonitril. Die Umsetzung erfolgt dann in gleicher Weise wie beim Verfahren (c) beschrieben. Für Verbindungen, in denen R₁ oder R₂ (Niederalkyl, Aryl oder Arylniederalkyl)thio bedeutet, sind geeignete reaktive Derivate die entsprechenden Disulfide, z.B. Dimethyldisulfid, Diphenyldisulfid oder Dibenzyldisulfid.

Ferner können Verbindungen der Formel I, worin Tetr 1- oder 2-Tetrazolyl bedeutet, in an sich bekannter Weise in Verbindungen der Formel I, worin Tetr für 1- oder 2-Tetrazolyl, welches in 5-Stellung substituiert ist, überführt werden. So können z.B. durch Alkylierung, etwa mit Niederalkylhalogeniden oder Phenylniederalkylhalogeniden, Niederalkyl- bzw. Phenylniederalkylgruppen eingeführt werden. Ferner können, z.B. durch Acylierung mit Niederalkansäurehalogeniden oder -anhydriden, Niederalkanoylgruppen eingeführt werden.

Verfahrensgemäss erhältliche freie Verbindungen der Formel I mit salzbildenden Eigenschaften können in an sich bekannter Weise in ihre Salze überführt werden, Verbindungen mit basischen Eigenschaften z.B. durch Behandeln mit Säuren oder geeigneten Derivaten davon, Verbindungen mit sauren Eigenschaften z.B. durch Behandeln mit Basen oder geeigneten Derivaten davon.

Erfindungsgemäss erhältliche Gemische von Isomeren können in an sich bekannter Weise in die einzelnen Isomeren aufgetrennt werden, Racemate z.B. durch Bilden von Salzen mit optisch reinen salzbildenden Reagentien und Auftrennen des so erhältlichen Diastereomerengemisches, z.B. mittels fraktionierter Kristallisation.

Die oben angeführten Reaktionen können unter an sich bekannten Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, je nach Art der Reaktion und/oder der Reaktionsteilnehmer bei erniedrigter, normaler oder erhöhter Temperatur, z.B. im Temperaturbereich von etwa -70°C bis etwa 200°C, vorzugsweise von etwa -20°C bis etwa 150°C, z.B. beim Siedepunkt des verwendeten Lösungsmittels, unter atmosphärischem Druck oder in einem geschlossenen Gefäss, gegebenenfalls unter Druck, und/oder in einer inerten Atmosphäre, z.B. unter einer Stickstoffatmosphäre.

Infolge der engen Beziehung zwischen den Verbindungen der Formel I in freier Form und in Form von Salzen sind vor- und nachstehend unter den freien Verbindungen bzw. ihren Salzen sinn- und zweckmässig gegebenenfalls auch die entsprechenden Salze bzw. freien Verbindungen zu verstehen.

Die Verbindungen, einschliesslich ihrer Salze, können auch in Form von Hydraten erhalten werden, oder ihre Kristalle können z.B. das zur Kristallisation verwendete Lösungsmittel einschliessen.

Im Verfahren der vorliegenden Erfindung werden vorzugsweise solche Ausgangsstoffe eingesetzt, die zu den eingangs als besonders wertvoll beschriebenen Verbindungen führen.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt, oder bei denen ein Ausgangsstoff unter den Reaktionsbedingungen gebildet oder in Form eines Derivates, z.B. eine Salzes davon, verwendet wird.

Die vorliegende Erfindung betrifft ebenfalls pharmazeutische Präparate, die als Wirkstoff eine der pharmakologisch wirksamen Verbindungen der Formel I enthalten. Besonders bevorzugt sind Präparate zur enteralen, insbesondere oralen, sowie zur parenteralen Verabreichung. Die Präparate enthalten den Wirkstoff allein oder vorzugsweise zusammen mit einem pharmazeutisch anwendbaren Trägermaterial. Die Dosierung des Wirkstoffs hängt von der zu behandelnden Krankheit, sowie von Spezies, deren Alter, Gewicht und individuellem Zustand, sowie von der Applikationsweise ab.

Die pharmazeutischen Präparate enthalten von etwa 0,1 % bis etwa 95 % des Wirkstoffs, wobei einzeldosierte Applikationsformen vorzugsweise von etwa 1 % bis etwa 90 % und nicht-einzeldosierte Applikationsformen vorzugsweise etwa 0,1 % bis etwa 20 % Wirkstoff aufweisen. Dosiseinheitsformen, wie Dragées, Tabletten oder Kapseln, enthalten von etwa 0,5 mg bis etwa 100 mg des Wirkstoffs.

Die pharmazeutischen Präparate der vorliegenden Erfindung werden in an sich bekannter Weise, z.B. mittels konventioneller Misch-, Granulier- Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls durch Zugabe von zusätzlichen Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken. z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol, oder Derivate davon.

Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Oral anwendbare pharmazeutische Zusammensetzungen sind ebenfalls Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten flüssigen Hilfsstoffen, wie fetten Oelen, Paraffinöl oder flüssigen Polyethylenglykolen, gelöst oder suspendiert, wobei ebenfalls Stabilisatoren zugefügt sein können.

Weitere orale Applikationsformen sind z.B. in üblicher Weise bereitete Sirups, die den Wirkstoff z.B. in suspendierter Form und in einer Konzentration von ca. 5 % bis 20 %, vorzugsweise ca. 10 % oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole.

Zu parenteralen Verabreichung eignen sich in erster Linie wässrige Lösungen eines Wirkstoffes in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Lösungen, wie sie z.B. für die parenterale Verabreichung verwendet werden, können auch als Infusionslösungen angewandt werden.

Die Erfindung betrifft auch eine Verbindung der Formel I zur Anwendung in einem Verfahren zur Behandlung des menschlichen oder tierischen Körpers, insbesondere bei der Behandlung von Krankheiten, die auf eine Hemmung des Enzyms Aromatase ansprechen; und die Verwendung von Verbindungen der Formel I zur Herstellung pharmazeutischer Präparate zur Behandlung der oben genannten Krankheitszustände. Die Verbindungen der vorliegenden Erfindung können prophylaktisch oder therapeutisch verabreicht werden, wobei man sie vorzugsweise in Form von pharmazeutischen Präparaten verwendet. Dabei wird bei einem Körpergewicht von etwa 70 kg eine tägliche Dosis von etwa 0,5 mg bis etwa 100 mg, vorzugsweise von etwa 1 mg bis etwa 20 mg, einer Verbindung der vorliegenden Erfindung verabreicht.

Die nachfolgenden Beispiele illustrieren die vorliegende Erfindung; Temperaturen werden in Grad Celsius angegeben. Folgende Abkürzungen werden verwendet: Ether = Diethylether; Essigester = Essigsäureethylester; THF = Tetrahydrofuran; Hexan = n-Hexan; DMSO = Dimethylsulfoxid; DMF = Dimethylformamid; DC = Dünnschichtchromatographie.

### Beispiel 1: 4-(2-Tetrazolyl)methyl-benzonitril und 4-(1-Tetrazolyl)methyl-benzonitril

Eine Lösung von 12 g 4-Brommethylbenzonitril in 400 ml Aceton wird nacheinander mit 6,3 g Tetrazol, 8,28 g Kaliumcarbonat und 0,675 g Kaliumiodid versetzt und anschliessend 19 h bei 40-45° gerührt. Das Reaktionsgemisch wird abgekühlt, filtriert und eingeengt. Der Rückstand wird zwischen CH₂Cl₂ und Wasser aufgetrennt. Die organische Phase wird mit Sole gewaschen und nach Trocknen über Natriumsulfat eingedampft. Die nachfolgende Säulenchromatographie (Kieselgel) liefert zuerst mit Toluol/Essigester (2:1) das 4-(2-Tetrazolyl)methyl-benzonitril [DC (Hexan/Essigester, 1:2) R_{f}=0,76); ¹H-NMR (CDCl₃): δ= 5,8 (2H,s), 7,4 und 7,7 (4H,m), 8,5 (1H,s) ppm], dann mit Essigester das 4-(1-Tetrazolyl)methyl-benzonitril [DC(Hexan/Essigester, 1:2), R_{f}=0,36); ¹H-NMR(CDCl₃): δ= 5,63 (2H,s), 7,4 und 7,7 (4H,m), 8,6 (1H,s) ppm].

### Beispiel 2: 4-[α-(4-Cyanophenyl)-(2-tetrazolyl)methyl]-benzonitril

Eine Lösung von 7,9 g Kalium-tert-butylat in 37 ml abs. DMF wird innerhalb von 30 min bei Raumtemperatur mit einer Lösung von 4,6 g 4-[(2-Tetrazolyl)methyl]-benzonitril (s. Beispiel 1) in 37 ml abs. DMF versetzt. Die dunkelgrüne Lösung wird nach 15 min Rühren innerhalb von weiteren 15 min mit einer Lösung von 3,8 g 4-Fluorbenzonitril in 37 ml abs. DMF versetzt. Das dunkle, rotbraune Reaktionsgemisch wird 1,5 h ausgerührt, und mit 30 ml CH₂Cl₂ verdünnt. Nach Abkühlen auf 0° wird mit 6N HCI neutral gestellt, und unter vermindertem Druck werden dann die Lösungsmittel eingedampft. Das erhaltene Harz wird zwischen CH₂Cl₂ und Wasser aufgeteilt, und die abgetrennte organische Lösung wird nochmals mit Sole gewaschen und über Natriumsulfat getrocknet. Die Reinigung erfolgt durch Säulenchromatographie [Kieselgel, Hexan/Essigester (2:1)] und anschliessende Kristallisation aus Ether; Smp. 110-112°; IR(CH₂Cl₂): 2220, 1605, 1497, 1410 cm⁻¹; ¹H-NMR(DMSO-d₆): δ= 7,48 und 7,92 (8H,m), 8,08 (1H,s), 9,18 (1H,s) ppm.

### Beispiel 3: 1-Cyano-4-(1-tetrazolyl)methyl-naphthalin und 1-Cyano-4-(2-tetrazolyl)methyl-naphthalin

Eine Lösung von 985 mg 1-Cyano-4-brommethyl-naphthalin in 20 ml Aceton wird mit 420 mg Tetrazol, 553 mg Kaliumcarbonat und 53 mg Kaliumiodid versetzt und während 3,5 h bei 45° gerührt. Nach dem Abkühlen des Reaktionsgemisches wird das Lösungsmittel abdestilliert und der Rückstand in Methylenchlorid gelöst. Nach dem Waschen der organischen Lösung mit Wasser und Sole wird diese über Natriumsulfat getrocknet und eingeengt. Die Trennung durch Säulenchromatographie ergibt zuerst durch Eluieren mit Toluol/Essigester (9:1) das 1-Cyano-4-(2-tetrazolyl)methyl-naphthalin, Smp. 124°, [IR(CH₂Cl₂): 3052,2225,1592,1516 cm⁻¹, ¹H-NMR(DMSO-d₆): δ= 6,62 (2H,s), 7,6 und 8,2 (2H,m), 7,83 (2H,m), 8,2 und 8,33 (2H,m), 9,02 (1H,s) ppm] und dann mit Toluol/Essigester (3:1) das 1-Cyano-4-(1-Tetrazolyl)methyl-naphthalin, Smp. 171° [IR(CH₂Cl₂): 3050,2227,1516,1482 cm⁻¹; ¹H-NMR(DMSO-d₆): δ= 6,36 (2H,s), 7,5 und 8,2 (2H,m), 7,85 (2H,m), 8,2 und 8,37 (2H,m), 9,6 (1H,s) ppm.

### Beispiel 4:

In analoger Weise wie in den vorhergehenden Beispielen beschrieben können die folgenden Verbindungen hergestellt werden:

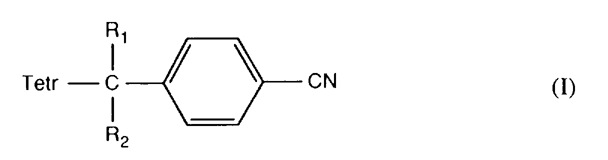

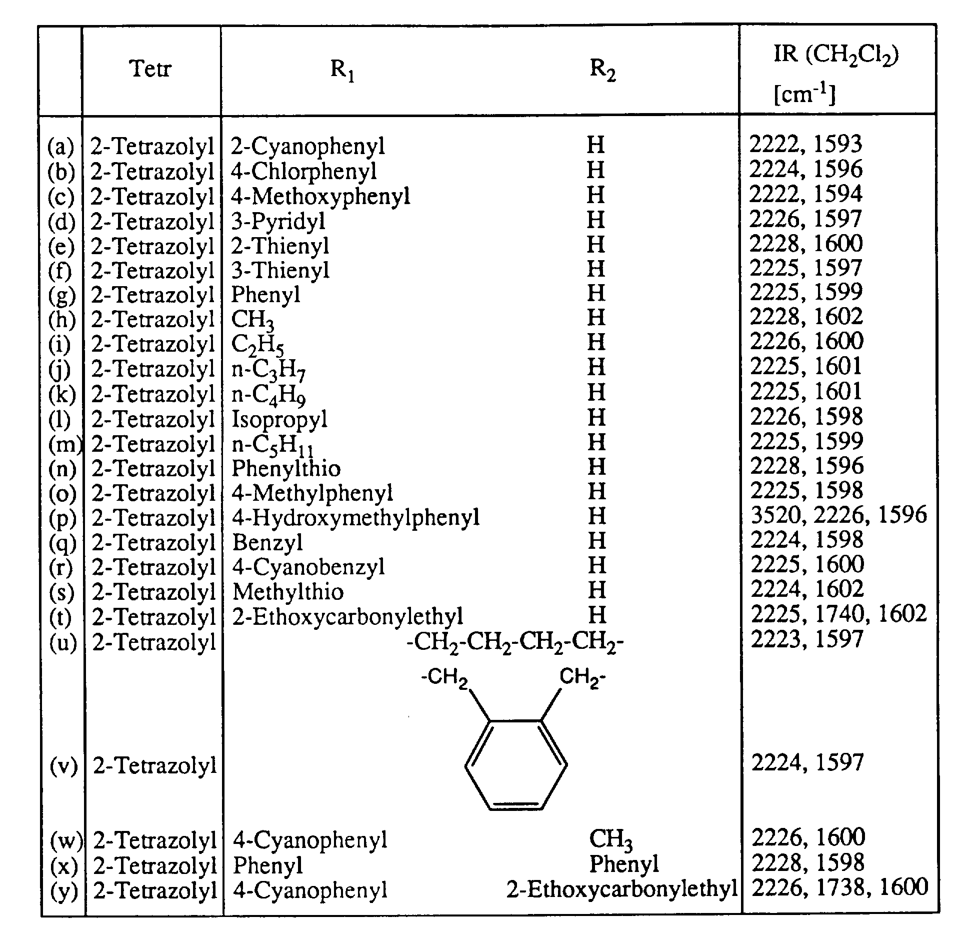

### Beispiel 5: 4-[α-(1-Methyl-1H-benzotriazol-6-yl)-(2-tetrazolyl)methyl]-benzonitril

1-Chlor-4-[α-(1-methyl-1H-benzotriazol-6-yl)-(2-tetrazolyl)methyl]-benzol wird mit Kupfer(I)cyanid umgesetzt, wobei man die Titelverbindung erhält.

Die Ausgangsverbindung wird wie folgt hergestellt:

### (a) 1-Methyl-1H-benzotriazol-6-carbonsäuremethylester

Benzotriazol-6-carbonsäuremethylester wird mit Methyliodid umgesetzt, wobei man die Titelverbindung (neben den isomeren 2-Methyl-2H- und 3-Methyl-3H-Verbindungen) erhält. Die Titelverbindung wird durch Chromatographie in reiner Form erhalten.

### (b) 1-Methyl-1H-benzotriazol-6-carbonsäure

Der Methylester (a) wird mit NaOH verseift, wobei man die Titelverbindung erhält.

Alternativ kann die Titelverbindung auch aus der Dimethyl-Verbindung von Beispiel 6a durch Oxidation mit KMnO₄ erhalten werden.

### (c) 6-(4-Chlorbenzoyl)-1-methyl-1H-benzotriazol

Die Carbonsäure (b) wird mit Thionylchlorid in das entsprechende Säurechlorid überführt und dann mit Chlorbenzol und AlCl₃ umgesetzt, wobei man die Titelverbindung erhält.

### (d) 6-[1-(4-Chlorphenyl)-1-hydroxymethyl]-1-methyl-1H-benzotriazol

Die Ketoverbindung (c) wird mit Natriumborhydrid reduziert, wobei man die Titelverbindung erhält.

Alternativ kann die Titelverbindung auch aus der Brommethyl-Verbindung von Beispiel 6b erhalten werden, und zwar durch (1) Oxidation mit Dimethylsulfoxid zum 6-Formyl-1-methyl-1H-benzotriazol und (2) Umsetzung der letzteren Verbindung mit 1-Chlor-4-chlormagnesiumbenzol (Grignardverbindung).

### (e) 6-[1-(4-Chlorphenyl)-1-chlormethyl]-1-methyl-1H-benzotriazol

Der Alkohol (d) wird mit Phosphortrichlorid umgesetzt, wobei man die Titelverbindung erhält.

### (f) 1-Chlor-4-[α-(1-methyl-1H-benzotriazol-6-yl)-(2-tetrazolyl)methyl]-benzol

Die Chlormethylverbindung (e) wird mit Tetrazol umgesetzt, wobei man die Titelverbindung erhält.

### Beispiel 6: 4-[α-(1-methyl-1H-benzotriazol-6-yl)-(2-tetrazolyl)methyl]-benzonitril

1-Methyl-6-(2-tetrazolyl)methyl-1H-benzotriazol wird mit Kalium-tert-butylat in abs. DMF mit 4-Fluorbenzonitril umgesetzt, wobei man die Titelverbindung erhält.

Die Ausgangsverbindung wird wie folgt hergestellt:

### (a) 1,6-Dimethyl-1H-benzotriazol

6-Methyl-benzotriazol wird mit Methyliodid umgesetzt, wobei man die Titelverbindung (neben den isomeren 2-Methyl-2H- und 3-Methyl-3H-Verbindungen) erhält. Die Titelverbindung wird durch Chromatographie in reiner Form erhalten.

### (b) 6-Brommethyl-1-methyl-1H-benzotriazol

Die Dimethyl-Verbindung (a) wird mit N-Bromsuccinimid umgesetzt, wobei man die Titelverbindung erhält.

### (c) 1-Methyl-6-(2-tetrazolyl)methyl-1H-benzotriazol

Die Brommethyl-Verbindung (b) wird mit Tetrazol umgesetzt, wobei man die Titelverbindung erhält.

### Beispiel 7: (a) 4-[α-(4-Cyanophenyl)-(1-tetrazolyl)methyl]-benzonitril und (b) 4-[α-(4-Cyanophenyl)-(2-tetrazolyl)methyl]-benzonitril

Eine Lösung von 197 mg 4-[α-(4-Cyanophenyl)-methylsulfonyloxymethyl]-benzonitril in 1 ml DMF wird mit 0,07 ml Triethylamin versetzt und die erhaltene orange Suspension nach Zugabe von 35 mg Tetrazol 2 h bei Raumtemperatur gerührt. Es werden weitere 0,07 ml Triethylamin hinzugegeben, und man rührt noch 5,75 h bei Raumtemperatur und 23 h bei 50°. Das Reaktionsgemisch wird mit Essigester verdünnt, viermal mit Sole gewaschen, über Natriumsulfat getrocknet und eingeengt. Durch Säulenchromatographie (SiO₂, 230-400 mesh, Toluol bis Toluol/Essigester 7:3) erhält man zuerst 4-[α-(4-Cyanophenyl)-(2-tetrazolyl)methyl]-benzonitril, Smp. 110-112° (wie Beispiel 2), und dann 4-[α-(4-Cyanophenyl)-(1-tetrazolyl)methyl]-benzonitril, DC (Toluol/Essigester 1:1) R_{f}=0,28, IR (CH₂Cl₂): 2220, 1605, 1497, 1460, 1405 cm⁻¹; ¹H-NMR (CDCl₃): δ = 7,47 und 7,94 (8H, m), 7,7 (1H, s), 9,6 (1H, s) ppm.

Das Ausgangsmaterial wird wie folgt hergestellt:

4,4'-Dicyanobenzophenon: Eine Lösung von 5,1 g 4,4'-Dibrombenzophenon in 90 ml DMF wird mit 8,13 g CuCN versetzt und während 13 h unter Rückfluss gerührt. Nach Abkühlen wird das Reaktionsgemisch mit Essigester verdünnt, zweimal mit 50 % wässriger Ethylendiamin-Lösung, zweimal mit Wasser und noch dreimal mit Sole gewaschen, getrocknet und eingeengt. Nach Säulenchromatographie (SiO₂, Toluol bis Toluol/Essigester 95:5) erhält man die kristalline Titelverbindung; DC (Toluol/Essigester 9:1): R_{f}= 0,34; IR (CH₂Cl₂): 2220, 1670, 1605, 1405 cm⁻¹.

4-[α-(4-Cyanophenyl)-hydroxymethyl]-benzonitril: Eine Suspension von 820 mg 4,4'-Di-cyanobenzophenon in 35 ml Methanol wird unter Eiskühlung mit 125 mg NaBH₄ versetzt und während 40 min gerührt. Dann wird das Reaktionsgemisch mit Essigsäure neutralisiert und im Vakuum eingeengt. Nach Aufteilen des Rückstandes zwischen CH₂Cl₂ und Wasser wird die organische Phase abgetrennt, mit Sole gewaschen, über Natriumsulfat getrocknet und eingeengt. Der kristalline Rückstand wird aus Essigester/Petrolether umkristallisiert; Smp. 158°; IR (CH₂Cl₂): 3580, 2220, 1605, 1495 cm⁻¹.

4-[α-(4-Cyanophenyl)-methylsulfonyloxymethyl]-benzonitril: Eine Suspension von 117 mg 4-[α-(4-Cyanophenyl)-hydroxymethyl]-benzonitril in 1 ml CH₂Cl₂ wird auf 0° gekühlt und nacheinander mit 0,078 ml Mesylchlorid (= Methansulfochlorid) und 0,070 ml Triethylamin versetzt. Nach weiteren 5 h bei 0° wird das Kühlbad entfernt und 2 h bei Raumtemperatur ausgerührt. Das Reaktionsgemisch wird in Essigester aufgenommen, mit kalter wässriger Natriumacetat-Lösung und Sole gewaschen, über Natriumsulfat getrocknet und eingeengt. Die Rohsubstanz wird ohne weitere Reinigung eingesetzt; IR (CH₂Cl₂): 2220, 1605, 1365, 1170 cm⁻¹.

### Beispiel 8:

Es werden 10000 Tabletten hergestellt, die je 5 mg Wirkstoff, z.B. eine der in den Beispielen 1-7 hergestellten Verbindungen, enthalten:

### Zusammensetzung:

| | |
|---|---|
| Wirkstoff | 50,00 g |
| Milchzucker | 2535,00 g |
| Maisstärke | 125,00 g |
| Polyethylenglykol 6.000 | 150,00 g |
| Magensiumstearat | 40,00 g |
| Gereinigtes Wasser | quantum satis |

Verfahren: Alle pulverigen Bestandteile werden durch ein Sieb mit einer Maschenweite von 0,6 mm gesiebt. Dann wird der Wirkstoff, der Milchzucker, das Magnesiumstearat und die Hälfte der Stärke in einem geeigneten Mischer vermischt. Die andere Hälfte der Stärke wird in 65 ml Wasser suspendiert und die entstandene Suspension zu einer siedenden Lösung des Polyethylenglykols in 260 ml Wasser gegeben. Die gebildete Paste wird dem Pulvergemisch zugesetzt und gegebenenfalls unter Zugabe von mehr Wasser granuliert. Das Granulat wird über Nacht bei 35°C getrocknet, durch ein Sieb mit 1,2 mm Maschenweite getrieben und zu Tabletten, welche eine Bruchrille aufweisen, gepresst.

### Beispiel 9:

Es werden 1000 Kapseln hergestellt, die je 10 mg Wirkstoff, z.B. eine der in den Beispielen 1-7 hergestellten Verbindungen, enthalten:

### Zusammensetzung:

| | |
|---|---|
| Wirkstoff | 10,00 g |
| Milchzucker | 207,00 g |
| Modifizierte Stärke | 80,00 g |
| Magnesiumstearat | 3,00 g |

Verfahren: Alle pulverigen Bestandteile werden durch ein Sieb mit einer Maschenweite von 0,6 mm gesiebt. Dann wird der Wirkstoff in einem geeigneten Mischer zuerst mit dem Magensiumstearat und dann mit dem Milchzucker und der Stärke bis zur Homogenität vermischt. Hartgelatinekapseln Nr. 2 werden mit je 300 mg der erhaltenen Mischung unter Verwendung einer Kapselfüllmaschine gefüllt.

## Patentansprüche (Patentansprüche für folgende(n) Vertragsstaat(en): AT, BE, CH, DE, DK, FR, GB, IT, LI, LU, NL, SE)

1. Verbindungen der Formel I, 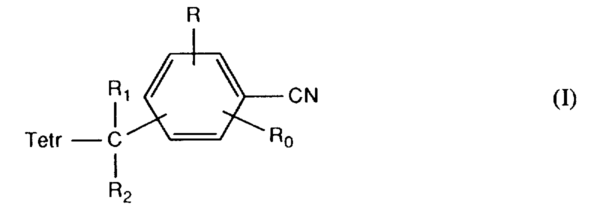 worin Tetr 1- oder 2-Tetrazolyl, welches unsubstituiert oder in 5-Stellung durch Niederalkyl, Phenylniederalkyl oder Niederalkanoyl substituiert ist, bedeutet; R₁ und R₂ unabhängig voneinander Wasserstoff; Niederalkyl, welches unsubstituiert oder durch Hydroxy, Niederalkoxy, Halogen, Carboxy, Niederalkoxycarbonyl, (Amino, Niederalkylamino oder Diniederalkylamino)-carbonyl oder Cyano substituiert ist; Niederalkenyl, Aryl, Hetaryl, Arylniederalkyl, C₃-C₆-Cycloalkyl, C₃-C₆-Cycloalkylniederalkyl, Niederalkylthio, Arylthio oder Arylniederalkylthio bedeuten; oder R₁ und R₂ zusammen C₄-C₆ geradkettiges Alkylen, welches unsubstituiert oder durch Niederalkyl substituiert ist, oder eine Gruppe -(CH₂)ₘ-1,2-Phenylen-(CH₂)ₙ-, worin m und n unabhängig voneinander 1 oder 2 bedeuten und 1,2-Phenylen unsubstituiert oder wie Phenyl gemäss der Definition von Aryl unten substituiert ist, oder Niederalkyliden, welches unsubstituiert oder durch Aryl mono- oder disubstituiert ist, bedeuten; und R und R₀ unabhängig voneinander Wasserstoff oder Niederalkyl bedeuten; oder R und R₀ zusammen, an benachbarten Kohlenstoffatomen des Benzolrings sitzend, eine Benzogruppe, die unsubstituiert oder wie Phenyl gemäss der Definition von Aryl unten substituiert ist, bedeuten; wobei in den obigen Definitionen Aryl jeweils Phenyl, welches unsubstituiert oder durch einen oder mehrere Substituenten aus der Gruppe bestehend aus Niederalkyl, Niederalkoxy, Hydroxy, Niederalkanoyloxy, Nitro, Amino, Halogen, Trifluormethyl, Carboxy, Niederalkoxycarbonyl, (Amino, Niederalkylamino oder Diniederalkylamino)-carbonyl, Cyano, Niederalkanoyl, Benzoyl, Niederalkylsulfonyl und (Amino, Niederalkylamino oder Diniederalkylamino)-sulfonyl substituiert ist, bedeutet; wobei in den obigen Definitionen Hetaryl einen aromatischen heterocyclischen Rest aus der Gruppe bestehend aus Pyrrolyl, Pyrazolyl, Imidazolyl, Triazolyl, Tetrazolyl, Furanyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl, Triazinyl, Indolyl, Isoindolyl, Benzimidazolyl, Benzotriazolyl, Benzofuranyl, Benzothienyl, Benzoxazolyl, Benzothiazolyl, Benzoxadiazolyl, Benzothiadiazolyl, Chinolyl und Isochinolyl, welcher unsubstituiert oder wie Phenyl gemäss der Definition von Aryl oben substituiert ist, bedeutet; und wobei in den oben genannten Definitionen "nieder" oder "Nieder" einen Rest bis und mit 7 Kohlenstoffatomen bedeutet; oder Salze davon.

2. Verbindungen der Formel I gemäss Anspruch 1, worin Tetr 1- oder 2-Tetrazolyl bedeutet; R₁ und R₂ unabhängig voneinander Wasserstoff; Niederalkyl, welches unsubstituiert oder durch Hydroxy, Niederalkoxy, Halogen, Carboxy, Niederalkoxycarbonyl, (Amino, Niederalkylamino oder Diniederalkylamino)-carbonyl oder Cyano substituiert ist; Niederalkenyl, Aryl, Hetaryl, Arylniederalkyl, C₃-C₆-Cycloalkyl, C₃-C₆-Cycloalkylniederalkyl, Niederalkylthio, Arylthio oder Arylniederalkylthio bedeuten; oder R₁ und R₂ zusammen C₄-C₆ geradkettiges Alkylen, welches unsubstituiert oder durch Niederalkyl substituiert ist, oder eine Gruppe -(CH₂)ₘ-1,2-Phenylen-(CH₂)ₙ-, worin m und n unabhängig voneinander 1 oder 2 bedeuten und 1,2-Phenylen unsubstituiert oder wie Phenyl gemäss der Definition von Aryl unten substituiert ist, oder Niederalkyliden, welches unsubstituiert oder durch Aryl mono- oder disubstituiert ist, bedeuten; und R und R₀ unabhängig voneinander Wasserstoff oder Niederalkyl bedeuten; oder R und R₀ zusammen, an benachbarten Kohlenstoffatomen des Benzolrings sitzend, eine Benzogruppe, die unsubstituiert oder wie Phenyl gemäss der Definition von Aryl unten substituiert ist, bedeuten; wobei in den obigen Definitionen Aryl jeweils Phenyl, welches unsubstituiert oder durch einen oder zwei Substituenten aus der Gruppe bestehend aus Niederalkyl, Niederalkoxy, Hydroxy, Niederalkanoyloxy, Nitro, Amino, Halogen, Trifluormethyl, Carboxy, Niederalkoxycarbonyl, (Amino, Niederalkylamino oder Diniederalkylamino)-carbonyl, Cyano, Niederalkanoyl, Benzoyl, Niederalkylsulfonyl und (Amino, Niederalkylamino oder Diniederalkylamino)-sulfonyl substituiert ist, bedeutet; wobei in den obigen Definitionen Hetaryl einen aromatischen heterocyclischen Rest aus der Gruppe bestehend aus Thienyl, Indolyl, Pyridyl, Furyl und Benzofuranyl, welcher unsubstituiert oder durch 1 bis 3 Substituenten aus der Gruppe bestehend aus Niederalkyl, Niederalkoxy, Cyano und Halogen substituiert ist, oder in 1-Stellung unsubstituiertes oder durch Niederalkyl, Hydroxy oder Niederalkoxy substituiertes Benzotriazolyl bedeutet; und wobei in den oben genannten Definitionen "nieder" oder "Nieder" einen Rest bis und mit 7 Kohlenstoffatomen bedeutet; oder Salze davon.

3. Verbindungen der Formel I gemäss Anspruch 1, worin Tetr 1- oder 2-Tetrazolyl bedeutet; R₁ und R₂ unabhängig voneinander Wasserstoff; Niederalkyl, welches unsubstituiert oder durch Niederalkoxycarbonyl substituiert ist; Phenyl, welches unsubstituiert oder durch Cyano, Halogen, Niederalkoxy, Niederalkyl oder Hydroxyniederalkyl substituiert ist; Thienyl, Pyridyl; 1-Niederalkyl-1H-benzotriazolyl; Phenylniederalkyl, welches im Phenylring unsubstituiert oder durch Cyano substituiert ist; Niederalkylthio oder Phenylthio bedeuten; oder R₁ und R₂ zusammen C₄-C₅ geradkettiges Alkylen oder eine Gruppe -CH₂-1,2-Phenylen-CH₂- bedeuten; und R und R₀ Wasserstoff bedeuten; oder R und R₀ zusammen, an benachbarten Kohlenstoffatomen des Benzolrings sitzend, eine Benzogruppe bedeuten; und wobei in den oben genannten Definitionen "nieder" oder "Nieder" einen Rest bis und mit 7 Kohlenstoffatomen bedeutet; oder Salze davon.

4. Verbindungen der Formel I gemäss Anspruch 1, worin Tetr 1- oder 2-Tetrazolyl bedeutet, R₁ Wasserstoff, Niederalkyl; Phenyl, welches unsubstituiert oder durch Cyano, Halogen, Niederalkoxy oder Niederalkyl substituiert ist; oder Phenylniederalkyl bedeutet; und R, R₀ und R₂ für Wasserstoff stehen; wobei in den oben genannten Definitionen "nieder" oder "Nieder" einen Rest bis und mit 7 Kohlenstoffatomen bedeutet; oder Salze davon.

5. Verbindungen der Formel I gemäss Anspruch 3, worin Tetr 1- oder 2-Tetrazolyl bedeutet; R₁ Wasserstoff; Niederalkyl, welches unsubstituiert oder durch Hydroxy, Niederalkoxy, Halogen, Carboxy, Niederalkoxycarbonyl, (Amino, Niederalkylamino oder Diniederalkylamino)-carbonyl oder Cyano substituiert ist; Niederalkenyl, Aryl, Hetaryl, Arylniederalkyl, C₃-C₆-Cycloalkyl, C₃-C₆-Cycloalkylniederalkyl, Niederalkylthio, Arylthio oder Arylniederalkylthio bedeutet; R₂ für Wasserstoff steht; oder R₁ und R₂ zusammen C₄-C₆ geradkettiges Alkylen, welches unsubstituiert oder durch Niederalkyl substituiert ist, oder eine Gruppe -(CH₂)ₘ-1,2-Phenylen-(CH₂)ₙ-, worin m und n unabhängig voneinander 1 oder 2 bedeuten und 1,2-Phenylen unsubstituiert oder wie Phenyl gemäss der Definition von Aryl unten substituiert ist, oder Niederalkyliden, welches unsubstituiert oder durch Aryl mono- oder disubstituiert ist, bedeuten; und R und Ro unabhängig voneinander Wasserstoff oder Niederalkyl bedeuten; oder R und Ro zusammen, an benachbarten Kohlenstoffatomen des Benzolrings sitzend, eine Benzogruppe, die unsubstituiert oder wie Phenyl gemäss der Definition von Aryl unten substituiert ist, bedeuten; wobei in den obigen Definitionen Aryl jeweils Phenyl, welches unsubstituiert oder durch einen Substituenten aus der Gruppe bestehend aus Niederalkyl, Niederalkoxy, Hydroxy, Niederalkanoyloxy, Nitro, Amino, Halogen, Trifluormethyl, Carboxy, Niederalkoxycarbonyl, (Amino, Niederalkylamino oder Diniederalkylamino)-carbonyl, Cyano, Niederalkanoyl, Benzoyl, Niederalkylsulfonyl und (Amino, Niederalkylamino oder Diniederalkylamino)-sulfonyl substituiert ist, bedeutet; wobei in den obigen Definitionen Hetaryl einen aromatischen heterocyclischen Rest aus der Gruppe bestehend aus Thienyl, Indolyl, Pyridyl, Furyl und Benzofuranyl, welcher unsubstituiert oder durch 1 oder 2 Substituenten aus der Gruppe bestehend aus Niederalkyl, Niederalkoxy, Cyano und Halogen substituiert ist, oder in 1-Stellung unsubstituiertes oder durch Niederalkyl, Hydroxy oder Niederalkoxy substituiertes Benzotriazolyl bedeutet; und wobei in den oben genannten Definitionen "nieder" oder "Nieder" einen Rest bis und mit 7 Kohlenstoffatomen bedeutet; oder Salze davon.

6. Verbindungen der Formel I gemäss Anspruch 4, worin Tetr 1- oder 2-Tetrazolyl bedeutet; R₁ Wasserstoff; Niederalkyl, welches unsubstituiert oder durch Niederalkoxycarbonyl substituiert ist; Phenyl, welches unsubstituiert oder durch Cyano, Halogen, Niederalkoxy, Niederalkyl oder Hydroxyniederalkyl substituiert ist; Thienyl, Pyridyl; Phenylniederalkyl, welches im Phenylring unsubstituiert oder durch Cyano substituiert ist; Niederalkylthio oder Phenylthio bedeutet; R₂ für Wasserstoff steht; oder R₁ und R₂ zusammen C₄-C₅ geradkettiges Alkylen oder eine Gruppe -CH₂-1,2-Phenylen-CH₂-bedeuten; und R und R₀ Wasserstoff bedeuten; oder R und R₀ zusammen, an benachbarten Kohlenstoffatomen des Benzolrings sitzend, eine Benzogruppe bedeuten; wobei in den oben genannten Definitionen "nieder" oder "Nieder" einen Rest bis und mit 7 Kohlenstoffatomen bedeutet; oder Salze davon.

7. 4-(2-Tetrazolyl)methyl-benzonitril gemäss Anspruch 1, oder ein pharmazeutisch verwendbares Salz davon.

8. 4-(1-Tetrazolyl)methyl-benzonitril gemäss Anspruch 1, oder ein pharmazeutisch verwendbares Salz davon.

9. 4-[α-(4-Cyanophenyl)-(2-tetrazolyl)methyl]-benzonitril gemäss Anspruch 1, oder ein pharmazeutisch verwendbares Salz davon.

10. 4-[α-(4-Cyanophenyl)-(1-tetrazolyl)methyl]-benzonitril gemäss Anspruch 1, oder ein pharmazeutisch verwendbares Salz davon.

11. Pharmzeutische Präparate enthaltend eine Verbindung gemäss einem der Ansprüche 1 bis 10 und mindestens ein pharmazeutisch verwendbares Trägermaterial.

12. Eine Verbindung gemäss einem der Ansprüche 1 bis 10 zur Anwendung in einem Verfahren zur prophylaktischen oder therapeutischen Behandlung des tierischen oder menschlichen Körpers.

13. Eine Verbindung gemäss einem der Ansprüche 1 bis 10 zur Verwendung bei der Behandlung von Krankheiten, die auf eine Hemmung des Enzyms Aromatase ansprechen.

14. Verwendung einer Verbindung gemäss einem der Ansprüche 1 bis 10 zur Herstellung pharmazeutischer Präparate.

15. Verwendung einer Verbindung gemäss einem der Ansprüche 1 bis 10 zur Herstellung pharmazeutischer Präparate für die Behandlung von Krankheiten, die auf eine Hemmung des Enzyms Aromatase ansprechen.

16. Verfahren zur Herstellung einer Verbindung der Formel I gemäss Anspruch 1, dadurch gekennzeichnet, dass man
(a) ein reaktives verestertes Derivat einer Verbindung der Formel II, 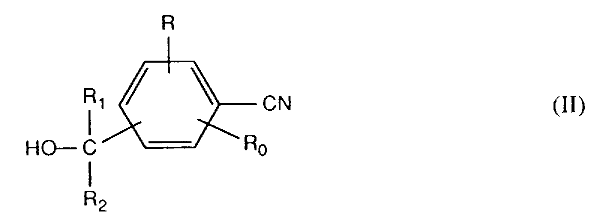 worin R, R₀, R₁ und R₂ wie unter Formel I definiert sind, mit einer Verbindung der Formel III,
Tetr-H (III)
worin Tetr 1- oder 2-Tetrazolyl bedeutet, oder einem N-geschützten Derivat davon, umsetzt, oder
(b) zur Herstellung von Verbindungen der Formel I, worin Tetr 1-Tetrazolyl bedeutet, in einer Verbindung der Formel IV, 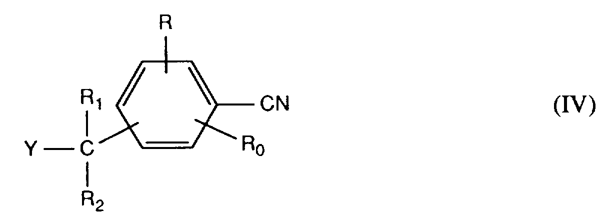 worin Y einen in 1-Tetrazolyl überführbaren Rest bedeutet und R, R₀, R₁ und R₂ wie unter Formel I definiert sind, Y in 1-Tetrazolyl umwandelt, oder
(c) eine Verbindung der Formel V, 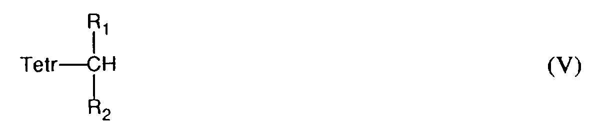 worin Tetr, R₁ und R₂ wie unter Formel I definiert sind, im basischen Medium mit einer Verbindung der Formel VI, 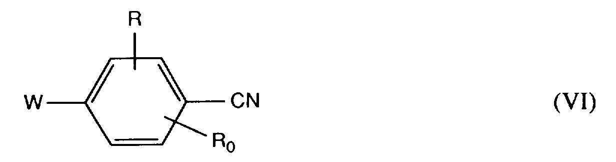 worin W eine Abgangsgruppe bedeutet und R und R₀ wie unter Formel I definiert sind, umsetzt, oder
(d) in einer Verbindung der Formel VII, 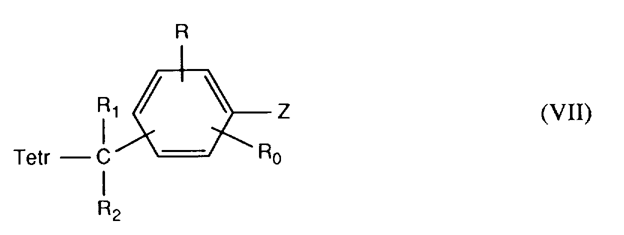 worin Z einen in Cyano überführbaren Rest bedeutet und Tetr, R, R₀, R₁ und R₂ wie unter Formel I definiert sind, den Rest Z in eine Cyanogruppe überführt; und/oder, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I in ein Salz umwandelt, und/oder ein erhaltenes Gemisch von isomeren Verbindungen der Formel I in die einzelnen Isomeren auftrennt.

## Patentansprüche (Patentansprüche für folgende(n) Vertragsstaat(en): ES, GR)

1. Verfahren zur Herstellung von Verbindungen der Formel I 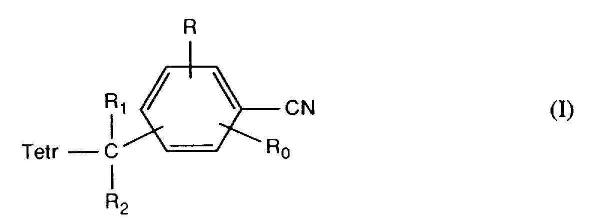 worin Tetr 1- oder 2-Tetrazolyl, welches unsubstituiert oder in 5-Stellung durch Niederalkyl, Phenylniederalkyl oder Niederalkanoyl substituiert ist, bedeutet; R₁ und R₂ unabhängig voneinander Wasserstoff; Niederalkyl, welches unsubstituiert oder durch Hydroxy, Niederalkoxy, Halogen, Carboxy, Niederalkoxycarbonyl, (Amino, Niederalkylamino oder Diniederalkylamino)-carbonyl oder Cyano substituiert ist; Niederalkenyl, Aryl, Hetaryl, Arylniederalkyl, C₃-C₆-Cycloalkyl, C₃-C₆-Cycloalkylniederalkyl, Niederalkylthio, Arylthio oder Arylniederalkylthio bedeuten; oder R₁ und R₂ zusammen C₄-C₆ geradkettiges Alkylen, welches unsubstituiert oder durch Niederalkyl substituiert ist, oder eine Gruppe -(CH₂)ₘ-1,2-Phenylen-(CH₂)ₙ-, worin m und n unabhängig voneinander 1 oder 2 bedeuten und 1,2-Phenylen unsubstituiert oder wie Phenyl gemäss der Definition von Aryl unten substituiert ist, oder Niederalkyliden, welches unsubstituiert oder durch Aryl mono- oder disubstituiert ist, bedeuten; und R und R₀ unabhängig voneinander Wasserstoff oder Niederalkyl bedeuten; oder R und R₀ zusammen, an benachbarten Kohlenstoffatomen des Benzolrings sitzend, eine Benzogruppe, die unsubstituiert oder wie Phenyl gemäss der Definition von Aryl unten substituiert ist, bedeuten; wobei in den obigen Definitionen Aryl jeweils Phenyl, welches unsubstituiert oder durch einen oder mehrere Substituenten aus der Gruppe bestehend aus Niederalkyl, Niederalkoxy, Hydroxy, Niederalkanoyloxy, Nitro, Amino, Halogen, Trifluormethyl, Carboxy, Niederalkoxycarbonyl, (Amino, Niederalkylamino oder Diniederalkylamino)-carbonyl, Cyano, Niederalkanoyl, Benzoyl, Niederalkylsulfonyl und (Amino, Niederalkylamino oder Diniederalkylamino)-sulfonyl substituiert ist, bedeutet; wobei in den obigen Definitionen Hetaryl einen aromatischen heterocyclischen Rest aus der Gruppe bestehend aus Pyrrolyl, Pyrazolyl, Imidazolyl, Triazolyl, Tetrazolyl, Furanyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl, Triazinyl, Indolyl, Isoindolyl, Benzimidazolyl, Benzotriazolyl, Benzofuranyl, Benzothienyl, Benzoxazolyl, Benzothiazolyl, Benzoxadiazolyl, Benzothiadiazolyl, Chinolyl und Isochinolyl, welcher unsubstituiert oder wie Phenyl gemäss der Definition von Aryl oben substituiert ist, bedeutet; und wobei in den oben genannten Definitionen "nieder" oder "Nieder" einen Rest bis und mit 7 Kohlenstoffatomen bedeutet; oder Salzen davon, dadurch gekennzeichnet, dass man
(a) ein reaktives verestertes Derivat einer Verbindung der Formel II, 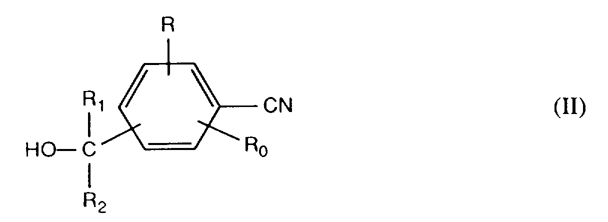 worin R, R₀, R₁ und R₂ wie unter Formel I definiert sind, mit einer Verbindung der Formel III,
Tetr-H (III)
worin Tetr 1- oder 2-Tetrazolyl bedeutet, oder einem N-geschützten Derivat davon, umsetzt, oder
(b) zur Herstellung von Verbindungen der Formel I, worin Tetr 1-Tetrazolyl bedeutet, in einer Verbindung der Formel IV, 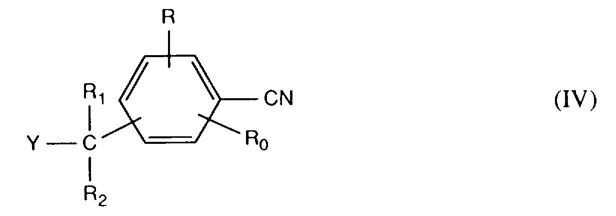 worin Y einen in 1-Tetrazolyl überführbaren Rest bedeutet und R, R₀, R₁ und R₂ wie unter Formel I definiert sind, Y in 1-Tetrazolyl umwandelt, oder
(c) eine Verbindung der Formel V, 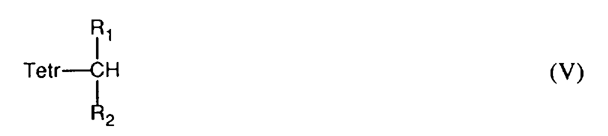 worin Tetr, R₁ und R₂ wie unter Formel I definiert sind, im basischen Medium mit einer Verbindung der Formel VI, 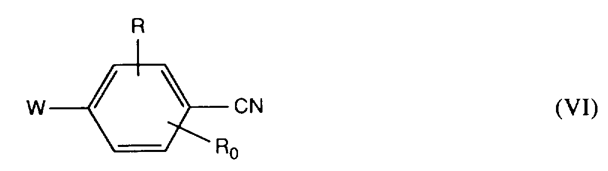 worin W eine Abgangsgruppe bedeutet und R und R₀ wie unter Formel I definiert sind, umsetzt, oder
(d) in einer Verbindung der Formel VII, 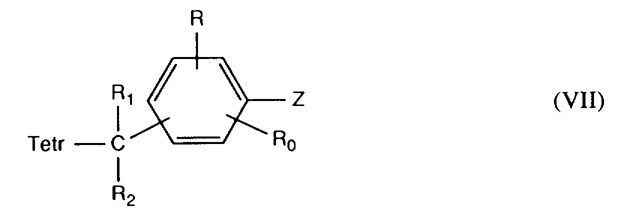 worin Z einen in Cyano überführbaren Rest bedeutet und Tetr, R, R₀, R₁ und R₂ wie unter Formel I definiert sind, den Rest Z in eine Cyanogruppe überführt; und/oder, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I in ein Salz umwandelt, und/oder ein erhaltenes Gemisch von isomeren Verbindungen der Formel I in die einzelnen Isomeren auftrennt.

2. Verfahren gemäss Anspruch 1 zur Herstellung von Verbindungen der Formel I, worin Tetr 1- oder 2-Tetrazolyl bedeutet; R₁ und R₂ unabhängig voneinander Wasserstoff; Niederalkyl, welches unsubstituiert oder durch Hydroxy, Niederalkoxy, Halogen, Carboxy, Niederalkoxycarbonyl, (Amino, Niederalkylamino oder Diniederalkylamino)carbonyl oder Cyano substituiert ist; Niederalkenyl, Aryl, Hetaryl, Arylniederalkyl, C₃-C₆-Cycloalkyl, C₃-C₆-Cycloalkylniederalkyl, Niederalkylthio, Arylthio oder Arylniederalkylthio bedeuten; oder R₁ und R₂ zusammen C₄-C₆ geradkettiges Alkylen, welches unsubstituiert oder durch Niederalkyl substituiert ist, oder eine Gruppe -(CH₂)ₘ-1,2-Phenylen-(CH₂)ₙ-, worin m und n unabhängig voneinander 1 oder 2 bedeuten und 1,2-Phenylen unsubstituiert oder wie Phenyl gemäss der Definition von Aryl unten substituiert ist, oder Niederalkyliden, welches unsubstituiert oder durch Aryl mono- oder disubstituiert ist, bedeuten; und R und R₀ unabhängig voneinander Wasserstoff oder Niederalkyl bedeuten; oder R und R₀ zusammen, an benachbarten Kohlenstoffatomen des Benzolrings sitzend, eine Benzogruppe, die unsubstituiert oder wie Phenyl gemäss der Definition von Aryl unten substituiert ist, bedeuten; wobei in den obigen Definitionen Aryl jeweils Phenyl, welches unsubstituiert oder durch einen oder zwei Substituenten aus der Gruppe bestehend aus Niederalkyl, Niederalkoxy, Hydroxy, Niederalkanoyloxy, Nitro, Amino, Halogen, Trifluormethyl, Carboxy, Niederalkoxycarbonyl, (Amino, Niederalkylamino oder Diniederalkylamino)-carbonyl, Cyano, Niederalkanoyl, Benzoyl, Niederalkylsulfonyl und (Amino, Niederalkylamino oder Diniederalkylamino)-sulfonyl substituiert ist, bedeutet; wobei in den obigen Definitionen Hetaryl einen aromatischen heterocyclischen Rest aus der Gruppe bestehend aus Thienyl, Indolyl, Pyridyl, Furyl und Benzofuranyl, welcher unsubstituiert oder durch 1 bis 3 Substituenten aus der Gruppe bestehend aus Niederalkyl, Niederalkoxy, Cyano und Halogen substituiert ist, oder in 1-Stellung unsubstituiertes oder durch Niederalkyl, Hydroxy oder Niederalkoxy substituiertes Benzotriazolyl bedeutet; und wobei in den oben genannten Definitionen "nieder" oder "Nieder" einen Rest bis und mit 7 Kohlenstoffatomen bedeutet; oder Salzen davon, dadurch gekennzeichnet, damm man entsprechend substituierte Ausgangsmaterialien verwendet.

3. Verfahren gemäss Anspruch 1 zur Herstellung von Verbindungen der Formel I, worin Tetr 1- oder 2-Tetrazolyl bedeutet; R₁ und R₂ unabhängig voneinander Wasserstoff; Niederalkyl, welches unsubstituiert oder durch Niederalkoxycarbonyl substituiert ist; Phenyl, welches unsubstituiert oder durch Cyano, Halogen, Niederalkoxy, Niederalkyl oder Hydroxyniederalkyl substituiert ist; Thienyl, Pyridyl; 1-Niederalkyl-1H-benzotriazolyl; Phenylniederalkyl, welches im Phenylring unsubstituiert oder durch Cyano substituiert ist; Niederalkylthio oder Phenylthio bedeuten; oder R₁ und R₂ zusammen C₄-C₅ geradkettiges Alkylen oder eine Gruppe -CH₂-1,2-Phenylen-CH₂- bedeuten; und R und R₀ Wasserstoff bedeuten; oder R und R₀ zusammen, an benachbarten Kohlenstoffatomen des Benzolrings sitzend, eine Benzogruppe bedeuten; und wobei in den oben genannten Definitionen "nieder" oder "Nieder" einen Rest bis und mit 7 Kohlenstoffatomen bedeutet; oder Salzen davon, dadurch gekennzeichnet, dass man entsprechend substituierte Ausgangsmaterialien verwendet.

4. Verfahren gemäss Anspruch 1 zur Herstellung von Verbindungen der Formel I, worin Tetr 1- oder 2-Tetrazolyl bedeutet, R₁ Wasserstoff, Niederalkyl; Phenyl, welches unsubstituiert oder durch Cyano, Halogen, Niederalkoxy oder Niederalkyl substituiert ist; oder Phenylniederalkyl bedeutet; und R, R₀ und R₂ für Wasserstoff stehen; wobei in den oben genannten Definitionen "nieder" oder "Nieder" einen Rest bis und mit 7 Kohlenstoffatomen bedeutet; oder Salzen davon, dadurch gekennzeichnet, dass man entsprechend substituierte Ausgangsmaterialien verwendet.

5. Verfahren gemäss Anspruch 1 zur Herstellung von Verbindungen der Formel I, worin Tetr 1- oder 2-Tetrazolyl bedeutet; R₁ Wasserstoff; Niederalkyl, welches unsubstituiert oder durch Hydroxy, Niederalkoxy, Halogen, Carboxy, Niederalkoxycarbonyl, (Amino, Niederalkylamino oder Diniederalkylamino)-carbonyl oder Cyano substituiert ist; Niederalkenyl, Aryl, Hetaryl, Arylniederalkyl, C₃-C₆-Cycloalkyl, C₃-C₆-Cycloalkylniederalkyl, Niederalkylthio, Arylthio oder Arylniederalkylthio bedeutet; R₂ für Wasserstoff steht; oder R₁ und R₂ zusammen C₄-C₆ geradkettiges Alkylen, welches unsubstituiert oder durch Niederalkyl substituiert ist, oder eine Gruppe -(CH₂)ₘ-1,2-Phenylen-(CH₂)ₙ-, worin m und n unabhängig voneinander 1 oder 2 bedeuten und 1,2-Phenylen unsubstituiert oder wie Phenyl gemäss der Definition von Aryl unten substituiert ist, oder Niederalkyliden, welches unsubstituiert oder durch Aryl mono- oder disubstituiert ist, bedeuten; und R und R₀ unabhängig voneinander Wasserstoff oder Niederalkyl bedeuten; oder R und R₀ zusammen, an benachbarten Kohlenstoffatomen des Benzolrings sitzend, eine Benzogruppe, die unsubstituiert oder wie Phenyl gemäss der Definition von Aryl unten substituiert ist, bedeuten; wobei in den obigen Definitionen Aryl jeweils Phenyl, welches unsubstituiert oder durch einen Substituenten aus der Gruppe bestehend aus Niederalkyl, Niederalkoxy, Hydroxy, Niederalkanoyloxy, Nitro, Amino, Halogen, Trifluormethyl, Carboxy, Niederalkoxycarbonyl, (Amino, Niederalkylamino oder Diniederalkylamino)carbonyl, Cyano, Niederalkanoyl, Benzoyl, Niederalkylsulfonyl und (Amino, Niederalkylamino oder Diniederalkylamino)-sulfonyl substituiert ist, bedeutet; wobei in den obigen Definitionen Hetaryl einen aromatischen heterocyclischen Rest aus der Gruppe bestehend aus Thienyl, Indolyl, Pyridyl, Furyl und Benzofuranyl, welcher unsubstituiert oder durch 1 oder 2 Substituenten aus der Gruppe bestehend aus Niederalkyl, Niederalkoxy, Cyano und Halogen substituiert ist, oder in 1-Stellung unsubstituiertes oder durch Niederalkyl, Hydroxy oder Niederalkoxy substituiertes Benzotriazolyl bedeutet; und wobei in den oben genannten Definitionen "nieder" oder "Nieder" einen Rest bis und mit 7 Kohlenstoffatomen bedeutet; oder Salzen davon, dadurch gekennzeichnet, dass man entsprechend substituierte Ausgangsmaterialien verwendet.

6. Verfahren gemäss Anspruch 4 zur Herstellung von Verbindungen der Formel I, worin Tetr 1- oder 2-Tetrazolyl bedeutet; R₁ Wasserstoff; Niederalkyl, welches unsubstituiert oder durch Niederalkoxycarbonyl substituiert ist; Phenyl, welches unsubstituiert oder durch Cyano, Halogen, Niederalkoxy, Niederalkyl oder Hydroxyniederalkyl substituiert ist; Thienyl, Pyridyl; Phenylniederalkyl, welches im Phenylring unsubstituiert oder durch Cyano substituiert ist; Niederalkylthio oder Phenylthio bedeutet; R₂ für Wasserstoff steht; oder R₁ und R₂ zusammen C₄-C₅ geradkettiges Alkylen oder eine Gruppe -CH₂-1,2-Phenylen-CH₂- bedeuten; und R und R₀ Wasserstoff bedeuten; oder R und R₀ zusammen, an benachbarten Kohlenstoffatomen des Benzolrings sitzend, eine Benzogruppe bedeuten; wobei in den oben genannten Definitionen "nieder" oder "Nieder" einen Rest bis und mit 7 Kohlenstoffatomen bedeutet; oder Salzen davon, dadurch gekennzeichnet, dass man entsprechend substituierte Ausgangsmaterialien verwendet.

7. Verfahren gemäss Anspruch 1 zur Herstellung einer Verbindung der Formel I mit dem Namen 4-(2-Tetrazolyl)methyl-benzonitril, oder von einem pharmazeutisch verwendbaren Salz davon, dadurch gekennzeichnet, dass man entsprechend substituierte Ausgangsmaterialien verwendet.

8. Verfahren gemäss Anspruch 1 zur Herstellung einer Verbindung der Formel I mit dem Namen 4-(1-Tetrazolyl)methyl-benzonitril, oder von einem pharmazeutisch verwendbaren Salz davon, dadurch gekennzeichnet, dass man entsprechend substituierte Ausgangsmaterialien verwendet.

9. Verfahren gemäss Anspruch 1 zur Herstellung einer Verbindung der Formel I mit dem Namen 4-[α-(4-Cyanophenyl)-(2-tetrazolyl)methyl]-benzonitril, oder von einem pharmazeutisch verwendbaren Salz davon, dadurch gekennzeichnet, dass man entsprechend substituierte Ausgangsmaterialien verwendet.

10. Verfahren gemäss Anspruch 1 zur Herstellung einer Verbindung der Formel I mit dem Namen 4-[α-(4-Cyanophenyl)-(1-tetrazolyl)methyl]-benzonitril gemäss Anspruch 1, oder von einem pharmazeutisch verwendbaren Salz davon, dadurch gekennzeichnet, dass man entsprechend substituierte Ausgangsmaterialien verwendet.

## Claims (Claims for the following Contracting State(s): AT, BE, CH, DE, DK, FR, GB, IT, LI, LU, NL, SE)

1. A compound of formula I 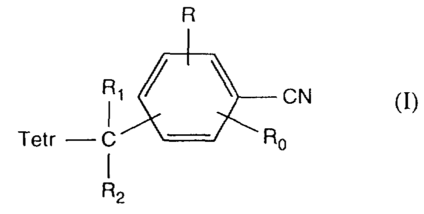 wherein Tetr is 1- or 2-tetrazolyl that is unsubstituted or substituted in the 5-position by lower alkyl, phenyl-lower alkyl or by lower alkanoyl; R₁ and R₂ are each independently of the other hydrogen; lower alkyl that is unsubstituted or substituted by hydroxy, lower alkoxy, halogen, carboxy, lower alkoxycarbonyl, (amino, lower alkylamino or di-lower alkylamino)-carbonyl or by cyano; lower alkenyl, aryl, hetaryl, aryl-lower alkyl, C₃-C₆-cycloalkyl, C3-C6cycloalkyl-lower alkyl, lower alkylthio, arylthio or aryl-lower alkylthio; or R₁ and R₂ together are C₄-C₆straight-chain alkylene that is unsubstituted or substituted by lower alkyl, or are a group -(CH₂)ₘ-1,2-phenylene-(CH₂)ₙ-, wherein m and n are each independently of the other 1 or 2 and 1,2-phenylene is unsubstituted or substituted in the same manner as phenyl in accordance with the definition of aryl below, or are lower alkylidene that is unsubstituted or mono- or di-substituted by aryl; and R and R₀ are each independently of the other hydrogen or lower alkyl; or R and R₀ together, located at adjacent carbon atoms of the benzene ring, form a benzo group that is unsubstituted or substituted in the same manner as phenyl in accordance with the definition of aryl below; and in the above definitions aryl is in each case phenyl that is unsubstituted or substituted by one or more substituents from the group consisting of lower alkyl, lower alkoxy, hydroxy, lower alkanoyloxy, nitro, amino, halogen, trifluoromethyl, carboxy, lower alkoxycarbonyl, (amino, lower alkylamino or di-lower alkylamino)-carbonyl, cyano, lower alkanoyl, benzoyl, lower alkylsulfonyl and (amino, lower alkylamino or di-lower alkylamino)-sulfonyl; and in the above definitions hetaryl is an aromatic heterocyclic radical from the group consisting of pyrrolyl, pyrazolyl, imidazolyl, triazolyl, tetrazolyl, furanyl, thienyl, isoxazolyl, oxazolyl, oxadiazolyl, isothiazolyl, thiazolyl, thiadiazolyl, pyridyl, pyridazinyl, pyrimidinyl, pyrazinyl, triazinyl, indolyl, isoindolyl, benzimidazolyl, benzotriazolyl, benzofuranyl, benzothienyl, benzoxazolyl, benzothiazolyl, benzoxadiazolyl, benzothiadiazolyl, quinolyl and isoquinolyl, which radical is unsubstituted or substituted in the same manner as phenyl in accordance with the definition of aryl above; and in the above definitions "-lower" or "lower" denotes a radical having up to and including 7 carbon atoms; or a salt thereof.

2. A compound of formula I according to claim 1, wherein Tetr is 1- or 2-tetrazolyl; R₁ and R₂ are each independently of the other hydrogen; lower alkyl that is unsubstituted or substituted by hydroxy, lower alkoxy, halogen, carboxy, lower alkoxycarbonyl, (amino, lower alkylamino or di-lower alkylamino)-carbonyl or by cyano; lower alkenyl, aryl, hetaryl, aryl-lower alkyl, C₃-C₆cycloalkyl, C₃-C₆vycloalkyl-lower alkyl, lower alkylthio, arylthio or aryl-lower alkylthio; or R₁ and R₂ together are C₄-C₆straight-chain alkylene that is unsubstituted or substituted by lower alkyl, or are a group -(CH₂)ₘ-1,2-phenylene-(CH₂)ₙ-, wherein m and n are each independently of the other 1 or 2 and 1,2-phenylene is unsubstituted or substituted in the same manner as phenyl in accordance with the definition of aryl below, or are lower alkylidene that is unsubstituted or mono- or di-substituted by aryl; and R and R₀ are each independently of the other hydrogen or lower alkyl; or R and R₀ together, located at adjacent carbon atoms of the benzene ring, form a benzo group that is unsubstituted or substituted in the same manner as phenyl in accordance with the definition of aryl below; and in the above definitions aryl is in each case phenyl that is unsubstituted or substituted by one or two substituents from the group consisting of lower alkyl, lower alkoxy, hydroxy, lower alkanoyloxy, nitro, amino, halogen, trifluoromethyl, carboxy, lower alkoxycarbonyl, (amino, lower alkylamino or di-lower alkylamino)-carbonyl, cyano, lower alkanoyl, benzoyl, lower alkylsulfonyl and (amino, lower alkylamino or di-lower alkylamino)-sulfonyl; and in the above definitions hetaryl is an aromatic heterocyclic radical from the group consisting of thienyl, indolyl, pyridyl, furyl and benzofuranyl, which radical is unsubstituted or substituted by from I to 3 substituents from the group consisting of lower alkyl, lower alkoxy, cyano and halogen, or is benzotriazolyl that in the 1-position is unsubstituted or substituted by lower alkyl, hydroxy or by lower alkoxy; and in the above definitions "-lower" or "lower" denotes a radical having up to and including 7 carbon atoms; or a salt thereof.

3. A compound of formula I according to claim 1, wherein Tetr is 1- or 2-tetrazolyl; R₁ and R₂ are each independently of the other hydrogen; lower alkyl that is unsubstituted or substituted by lower alkoxycarbonyl; phenyl that is unsubstituted or substituted by cyano, halogen, lower alkoxy, lower alkyl or by hydroxy-lower alkyl; thienyl, pyridyl; 1-lower alkyl-1H-benzotriazolyl; phenyl-lower alkyl that in the phenyl ring is unsubstituted or substituted by cyano; lower alkylthio or phenylthio; or R₁ and R₂ together are C₄-C₅straight-chain alkylene or a group -CH₂-1,2-phenylene-CH₂-; and R and R₀ are hydrogen; or R and R₀ together, located at adjacent carbon atoms of the benzene ring, form a benzo group; and in the above definitions "-lower" or "lower" denotes a radical having up to and including 7 carbon atoms; or a salt thereof.

4. A compound of formula I according to claim 1, wherein Tetr is 1- or 2-tetrazolyl, R₁ is hydrogen, lower alkyl; phenyl that is unsubstituted or substituted by cyano, halogen, lower alkoxy or by lower alkyl; or phenyl-lower alkyl; and R, R₀ and R₂ are hydrogen, and in the above definitions "-lower" or "lower" denotes a radical having up to and including 7 carbon atoms; or a salt thereof.

5. A compound of formula I according to claim 3, wherein Tetr is 1- or 2-tetrazolyl; R₁ is hydrogen; lower alkyl that is unsubstituted or substituted by hydroxy, lower alkoxy, halogen, carboxy, lower alkoxycarbonyl, (amino, lower alkylamino or di-lower alkylamino)-carbonyl or by cyano; lower alkenyl, aryl, hetaryl, aryl-lower alkyl, C₃-C₆cycloalkyl, C₃-C₆cycloalkyl-lower alkyl, lower alkylthio, arylthio or aryl-lower alkylthio; R₂ is hydrogen; or R₁ and R₂ together are C₄-C₆straight-chain alkylene that is unsubstituted or substituted by lower alkyl, or are a group -(CH₂)ₘ-1,2-phenylene-(CH₂)ₙ-, wherein m and n are each independently of the other 1 or 2 and 1,2-phenylene is unsubstituted or substituted in the same manner as phenyl in accordance with the definition of aryl below, or are lower alkylidene that is unsubstituted or mono- or di-substituted by aryl; and R and R₀ are each independently of the other hydrogen or lower alkyl; or R and R₀ together, located at adjacent carbon atoms of the benzene ring, form a benzo group that is unsubstituted or substituted in the same manner as phenyl in accordance with the definition of aryl below; and in the above definitions aryl is in each case phenyl that is unsubstituted or substituted by a substituent from the group consisting of lower alkyl, lower alkoxy, hydroxy, lower alkanoyloxy, nitro, amino, halogen, trifluoromethyl, carboxy, lower alkoxycarbonyl, (amino, lower alkylamino or di-lower alkylamino)-carbonyl, cyano, lower alkanoyl, benzoyl, lower alkylsulfonyl and (amino, lower alkylamino or di-lower alkylamino)sulfonyl; and in the above definitions hetaryl is an aromatic heterocyclic radical from the group consisting of thienyl, indolyl, pyridyl, furyl and benzofuranyl, which radical is unsubstituted or substituted by I or 2 substituents from the group consisting of lower alkyl, lower alkoxy, cyano and halogen, or is benzotriazolyl that in the 1-position is unsubstituted or substituted by lower alkyl, hydroxy or by lower alkoxy; and in the above definitions "-lower" or "lower" denotes a radical having up to and including 7 carbon atoms; or a salt thereof.

6. A compound of formula I according to claim 4, wherein Tetr is 1- or 2-tetrazolyl; R₁ is hydrogen; lower alkyl that is unsubstituted or substituted by lower alkoxycarbonyl; phenyl that is unsubstituted or substituted by cyano, halogen, lower alkoxy, lower alkyl or by hydroxy-lower alkyl; thienyl, pyridyl; phenyl-lower alkyl that in the phenyl ring is unsubstituted or substituted by cyano; lower alkylthio or phenylthio; R₂ is hydrogen; or R₁ and R₂ together are C₄-C₅straight-chain alkylene or a group -CH₂-1,2-phenylene-CH₂-; and R and R₀ are hydrogen; or R and R₀ together, located at adjacent carbon atoms of the benzene ring, form a benzo group; and in the above definitions "-lower" or "lower" denotes a radical having up to and including 7 carbon atoms; or a salt thereof.

7. 4-(2-Tetrazolyl)methyl-benzonitrile according to claim 1, or a pharmaceutically acceptable salt thereof.

8. 4-(1-Tetrazolyl)methyl-benzonitrile according to claim 1, or a pharmaceutically acceptable salt thereof.

9. 4-[α-(4-Cyanophenyl)-(2-tetrazolyl)methyl]-benzonitrile according to claim 1, or a pharmaceutically acceptable salt thereof.

10. 4-[α-(4-Cyanophenyl)-(1-tetrazolyl)methyl]-benzonitrile according to claim 1, or a pharmaceutically acceptable salt thereof.

11. A pharmaceutical preparation comprising a compound according to any one of claims 1 to 10 and at least one pharmaceutically acceptable carrier.

12. A compound according to any one of claims 1 to 10 for use in a method for the prophylactic or therapeutic treatment of the animal or human body.

13. A compound according to any one of claims 1 to 10 for use in the treatment of diseases that respond to inhibition of the enzyme aromatase.

14. The use of a compound according to any one of claims 1 to 10 for the manufacture of pharmaceutical preparations.

15. The use of a compound according to any one of claims 1 to 10 for the manufacture of pharmaceutical preparations for the treatment of diseases that respond to inhibition of the enzyme aromatase.

16. A process for the preparation of a compound of formula I according to claim 1, which process comprises:
(a) reacting a reactive esterified derivative of a compound of formula II 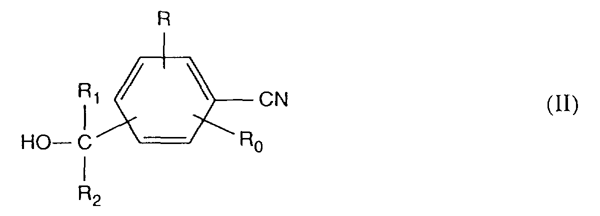 wherein R, R₀, R₁ and R₂ are as defined under formula I, with a compound of formula III
Tetr-H (III)
wherein Tetr is 1- or 2-tetrazolyl, or with an N-protected derivative thereof, or
(b) for the preparation of compounds of formula I wherein Tetr is 1-tetrazolyl, in a compound of formula IV 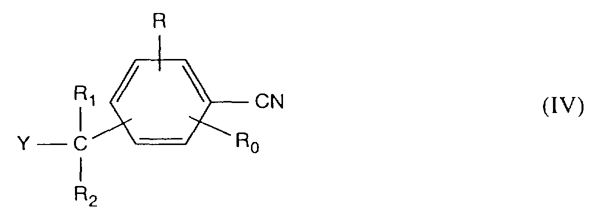 wherein Y is a radical that can be converted into 1-tetrazolyl and R, R₀, R₁ and R₂ are as defined under formula I, converting Y into 1-tetrazolyl, or
(c) reacting a compound of formula V 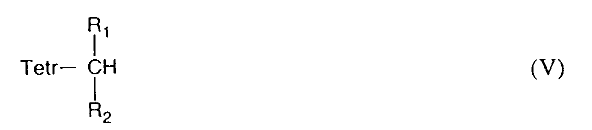 wherein Tetr, R₁ and R₂ are as defined under formula I, in a basic medium with a compound of formula VI, 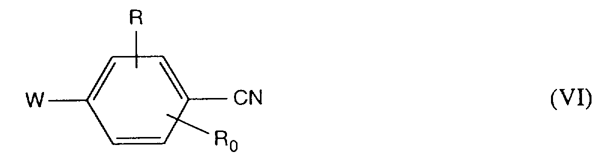 wherein W is a leaving group and R and R₀ are as defined under formula I, or
(d) in a compound of formula VII, 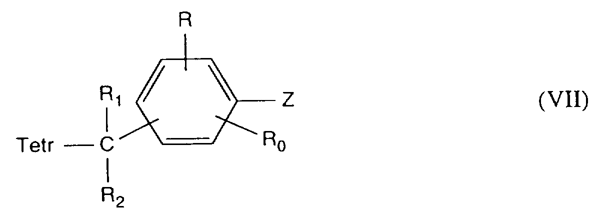 wherein Z is a radical that can be converted into cyano and Tetr, R, R₀, R₁ and R₂ are as defined under formula I, converting the radical Z into a cyano group; and/or, if desired, converting a resulting compound of formula I into a different compound of formula I, and/or, if desired, converting a resulting salt into the free compound or into a different salt, and/or, if desired, converting a resulting free compound of formula I into a salt, and/or separating a resulting mixture of isomeric compounds of formula I into the individual isomers.

## Claims (Claims for the following Contracting State(s): ES, GR)

1. A process for the preparation of a compound of formula I 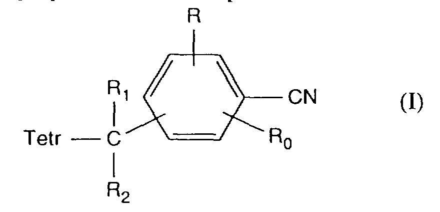 wherein Tetr is 1- or 2-tetrazolyl that is unsubstituted or substituted in the 5-position by lower alkyl, phenyl-lower alkyl or by lower alkanoyl; R₁ and R₂ are each independently of the other hydrogen; lower alkyl that is unsubstituted or substituted by hydroxy, lower alkoxy, halogen, carboxy, lower alkoxycarbonyl, (amino, lower alkylamino or di-lower alkylamino)-carbonyl or by cyano; lower alkenyl, aryl, hetaryl, aryl-lower alkyl, C₃-C₆-cycloalkyl, C₃-C₆cycloalkyl-lower alkyl, lower alkylthio, arylthio or aryl-lower alkylthio; or R₁ and R₂ together are C₄-C₆straight-chain alkylene that is unsubstituted or substituted by lower alkyl, or are a group -(CH₂)ₘ-1,2-phenylene-(CH₂)ₙ-, wherein m and n are each independently of the other I or 2 and 1,2-phenylene is unsubstituted or substituted in the same manner as phenyl in accordance with the definition of aryl below, or are lower alkylidene that is unsubstituted or mono- or di-substituted by aryl; and R and R₀ are each independently of the other hydrogen or lower alkyl; or R and R₀ together, located at adjacent carbon atoms of the benzene ring, form a benzo group that is unsubstituted or substituted in the same manner as phenyl in accordance with the definition of aryl below; and in the above definitions aryl is in each case phenyl that is unsubstituted or substituted by one or more substituents from the group consisting of lower alkyl, lower alkoxy, hydroxy, lower alkanoyloxy, nitro, amino, halogen, trifluoromethyl, carboxy, lower alkoxycarbonyl, (amino, lower alkylamino or di-lower alkylamino)-carbonyl, cyano, lower alkanoyl, benzoyl, lower alkylsulfonyl and (amino, lower alkylamino or di-lower alkylamino)-sulfonyl; and in the above definitions hetaryl is an aromatic heterocyclic radical from the group consisting of pyrrolyl, pyrazolyl, imidazolyl, triazolyl, tetrazolyl, furanyl, thienyl, isoxazolyl, oxazolyl, oxadiazolyl, isothiazolyl, thiazolyl, thiadiazolyl, pyridyl, pyridazinyl, pyrimidinyl, pyrazinyl, triazinyl, indolyl, isoindolyl, benzimidazolyl, benzotriazolyl, benzofuranyl, benzothienyl, benzoxazolyl, benzothiazolyl, benzoxadiazolyl, benzothiadiazolyl, quinolyl and isoquinolyl, which radical is unsubstituted or substituted in the same manner as phenyl in accordance with the definition of aryl above; and in the above definitions "-lower" or "lower" denotes a radical having up to and including 7 carbon atoms; or a salt thereof, which process comprises
(a) reacting a reactive esterified derivative of a compound of formula II 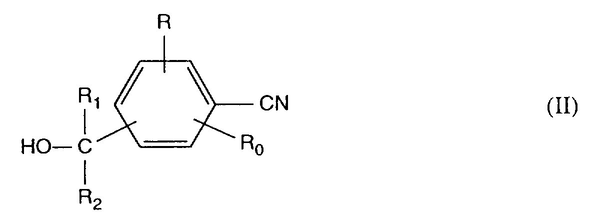 wherein R, R₀, R₁ and R₂ are as defined under formula I, with a compound of formula III
Tetr-H (III)
wherein Tetr is 1- or 2-tetrazolyl, or with an N-protected derivative thereof, or
(b) for the preparation of compounds of formula I wherein Tetr is 1-tetrazolyl, in a compound of formula IV 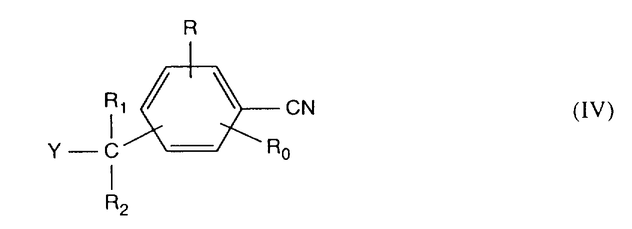 wherein Y is a radical that can be converted into 1-tetrazolyl and R, R₀, R₁ and R₂ are as defined under formula I, converting Y into 1-tetrazolyl, or
(c) reacting a compound of formula V 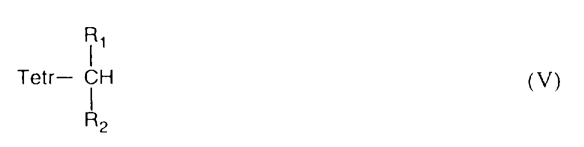 wherein Tetr, R₁ and R₂ are as defined under formula I, in a basic medium with a compound of formula VI 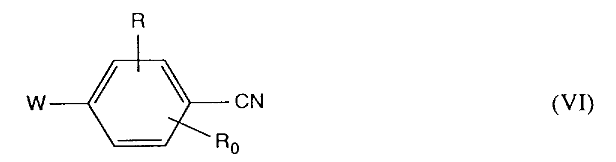 wherein W is a leaving group and R and R₀ are as defined under formula I, or
(d) in a compound of formula VII 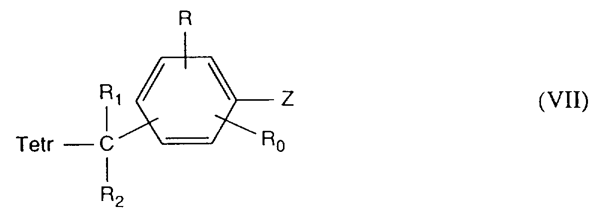 wherein Z is a radical that can be converted into cyano and Tetr, R, R₀, R₁ and R₂ are as defined under formula I, converting the radical Z into a cyano group; and/or, if desired, converting a resulting compound of formula I into a different compound of formula I, and/or, if desired, converting a resulting salt into the free compound or into a different salt, and/or, if desired, converting a resulting free compound of formula I into a salt, and/or separating a resulting mixture of isomeric compounds of formula I into the individual isomers.

2. A process according to claim I for the preparation of a compound of formula I wherein Tetr is I - or 2-tetrazolyl; R₁ and R₂ are each independently of the other hydrogen; lower alkyl that is unsubstituted or substituted by hydroxy, lower alkoxy, halogen, carboxy, lower alkoxycarbonyl, (amino, lower alkylamino or di-lower alkylamino)-carbonyl or by cyano; lower alkenyl, aryl, hetaryl, aryl-lower alkyl, C₃-C₆cycloalkyl, C₃-C₆cycloalkyl-lower alkyl, lower alkylthio, arylthio or aryl-lower alkylthio; or R₁ and R₂ together are C₄-C₆straight-chain alkylene that is unsubstituted or substituted by lower alkyl, or are a group -(CH₂)ₘ-1,2-phenylene-(CH₂)ₙ-, wherein m and n are each independently of the other I or 2 and 1,2-phenylene is unsubstituted or substituted in the same manner as phenyl in accordance with the definition of aryl below, or are lower alkylidene that is unsubstituted or mono- or di-substituted by aryl; and R and R₀ are each independently of the other hydrogen or lower alkyl; or R and R₀ together, located at adjacent carbon atoms of the benzene ring, form a benzo group that is unsubstituted or substituted in the same manner as phenyl in accordance with the definition of aryl below; and in the above definitions aryl is in each case phenyl that is unsubstituted or substituted by one or two substituents from the group consisting of lower alkyl, lower alkoxy, hydroxy, lower alkanoyloxy, nitro, amino, halogen, trifluoromethyl, carboxy, lower alkoxycarbonyl, (amino, lower alkylamino or di-lower alkylamino)-carbonyl, cyano, lower alkanoyl, benzoyl, lower alkylsulfonyl and (amino, lower alkylamino or di-lower alkylamino)sulfonyl; and in the above definitions hetaryl is an aromatic heterocyclic radical from the group consisting of thienyl, indolyl, pyridyl, furyl and benzofuranyl, which radical is unsubstituted or substituted by from 1 to 3 substituents from the group consisting of lower alkyl, lower alkoxy, cyano and halogen, or is benzotriazolyl that in the 1-position is unsubstituted or substituted by lower alkyl, hydroxy or by lower alkoxy; and in the above definitions "-lower" or "lower" denotes a radical having up to and including 7 carbon atoms; or a salt thereof, in which process appropriately substituted starting materials are used.

3. A process according to claim 1 for the preparation of a compound of formula I wherein Tetr is 1- or 2-tetrazolyl; R₁ and R₂ are each independently of the other hydrogen; lower alkyl that is unsubstituted or substituted by lower alkoxycarbonyl; phenyl that is unsubstituted or substituted by cyano, halogen, lower alkoxy, lower alkyl or by hydroxy-lower alkyl; thienyl, pyridyl; 1-lower alkyl-1H-benzotriazolyl; phenyl-lower alkyl that in the phenyl ring is unsubstituted or substituted by cyano; lower alkylthio or phenylthio; or R₁ and R₂ together are C₄-C₅straight-chain alkylene or a group -CH₂-1,2-phenylene-CH₂-; and R and R₀ are hydrogen; or R and R₀ together, located at adjacent carbon atoms of the benzene ring, form a benzo group; and in the above definitions "-lower" or "lower" denotes a radical having up to and including 7 carbon atoms; or a salt thereof, in which process appropriately substituted starting materials are used.

4. A process according to claim 1 for the preparation of a compound of formula I wherein Tetr is 1- or 2-tetrazolyl, R₁ is hydrogen, lower alkyl; phenyl that is unsubstituted or substituted by cyano, halogen, lower alkoxy or by lower alkyl; or phenyl-lower alkyl; and R, R₀ and R₂ are hydrogen, and in the above definitions "-lower" or "lower" denotes a radical having up to and including 7 carbon atoms; or a salt thereof, in which process appropriately substituted starting materials are used.

5. A process according to claim 1 for the preparation of a compound of formula I wherein Tetr is 1- or 2-tetrazolyl; R₁ is hydrogen; lower alkyl that is unsubstituted or substituted by hydroxy, lower alkoxy, halogen, carboxy, lower alkoxycarbonyl, (amino, lower alkylamino or di-lower alkylamino)-carbonyl or by cyano; lower alkenyl, aryl, hetaryl, aryl-lower alkyl, C₃-C₆cycloalkyl, C₃-C₆cycloalkyl-lower alkyl, lower alkylthio, arylthio or aryl-lower alkylthio; R₂ is hydrogen; or R₁ and R₂ together are C₄-C₆straight-chain alkylene that is unsubstituted or substituted by lower alkyl, or are a group -(CH₂)ₘ-1,2-phenylene-(CH₂)ₙ-, wherein m and n are each independently of the other 1 or 2 and 1,2-phenylene is unsubstituted or substituted in the same manner as phenyl in accordance with the definition of aryl below, or are lower alkylidene that is unsubstituted or mono- or di-substituted by aryl; and R and R₀ are each independently of the other hydrogen or lower alkyl; or R and R₀ together, located at adjacent carbon atoms of the benzene ring, form a benzo group that is unsubstituted or substituted in the same manner as phenyl in accordance with the definition of aryl below; and in the above definitions aryl is in each case phenyl that is unsubstituted or substituted by a substituent from the group consisting of lower alkyl, lower alkoxy, hydroxy, lower alkanoyloxy, nitro, amino, halogen, trifluoromethyl, carboxy, lower alkoxycarbonyl, (amino, lower alkylamino or di-lower alkylamino)-carbonyl, cyano, lower alkanoyl, benzoyl, lower alkylsulfonyl and (amino, lower alkylamino or di-lower alkylamino)-sulfonyl; and in the above definitions hetaryl is an aromatic heterocyclic radical from the group consisting of thienyl, indolyl, pyridyl, furyl and benzofuranyl, which radical is unsubstituted or substituted by 1 or 2 substituents from the group consisting of lower alkyl, lower alkoxy, cyano and halogen, or is benzotriazolyl that in the 1-position is unsubstituted or substituted by lower alkyl, hydroxy or by lower alkoxy; and in the above definitions "-lower" or "lower" denotes a radical having up to and including 7 carbon atoms; or a salt thereof, in which process appropriately substituted starting materials are used.

6. A process according to claim 4 for the preparation of a compound of formula I wherein Tetr is 1- or 2-tetrazolyl; R₁ is hydrogen; lower alkyl that is unsubstituted or substituted by lower alkoxycarbonyl; phenyl that is unsubstituted or substituted by cyano, halogen, lower alkoxy, lower alkyl or by hydroxy-lower alkyl; thienyl, pyridyl; phenyl-lower alkyl that in the phenyl ring is unsubstituted or substituted by cyano; lower alkylthio or phenylthio; R₂ is hydrogen; or R₁ and R₂ together are C₄-C₅straight-chain alkylene or a group -CH₂-1,2-phenylene-CH₂-; and R and R₀ are hydrogen; or R and R₀ together, located at adjacent carbon atoms of the benzene ring, form a benzo group; and in the above definitions "-lower" or "lower" denotes a radical having up to and including 7 carbon atoms; or a salt thereof, in which process appropriately substituted starting materials are used.

7. A process according to claim 1 for the preparation of a compound of formula I having the name 4-(2-tetrazolyl)methyl-benzonitrile, or a pharmaceutically acceptable salt thereof, in which process appropriately substituted starting materials are used.

8. A process according to claim 1 for the preparation of a compound of formula I having the name 4-(1-tetrazolyl)methyl-benzonitrile, or a pharmaceutically acceptable salt thereof, in which process appropriately substituted starting materials are used.

9. A process according to claim 1 for the preparation of a compound of formula I having the name 4-[α-(4-cyanophenyl)-(2-tetrazolyl)methyl]-benzonitrile, or a pharmaceutically acceptable salt thereof, in which process appropriately substituted starting materials are used.

10. A process according to claim 1 for the preparation of a compound of formula I having the name 4-[α-(4-cyanophenyl)-(1-tetrazolyl)methyl]-benzonitrile according to claim 1, or a pharmaceutically acceptable salt thereof, in which process appropriately substituted starting materials are used.

## Revendications (Revendications pour l'(les) Etat(s) contractant(s) suivant(s): AT, BE, CH, DE, DK, FR, GB, IT, LI, LU, NL, SE)

1. Composés de formule I 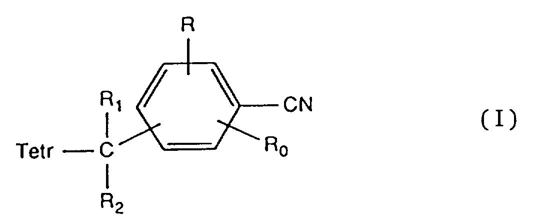 dans laquelle Tetr représente un groupe 1- ou 2-tétrazolyle qui est non substitué ou substitué en position 5 par un groupe alkyle inférieur, phényl-alkyle inférieur ou alcanoyle inférieur ; R₁ et R₂ représentent chacun, indépendamment l'un de l'autre, l'hydrogène ; un groupe alkyle inférieur non substitué ou substitué par des groupes hydroxy, alcoxy inférieur, des halogènes, des groupes carboxy, (alcoxy inférieur)-carbonyle, aminocarbonyle, (alkyle inférieur)aminocarbonyle ou di-(alkyle inférieur)aminocarbonyle ou cyano ; un groupe alcényle inférieur, aryle, hétéroaryle, aryl-alkyle inférieur, cycloalkyle en C3-C6, (cycloalkyle en C3-C6)alkyle inférieur, alkylthio inférieur, arylthio ou aryl-(alkyle inférieur)thio ; ou bien R₁ et R₂ forment ensemble un groupe alkylène à chaîne droite en C4-C6, non substitué ou substitué par un groupe alkyle inférieur, ou un groupe -(CH₂)ₘ-1,2-phénylène-(CH₂)ₙ- dans lequel m et n sont égaux chacun, indépendamment l'un de l'autre, à 1 ou 2 et la partie 1,2-phénylène est non substituée ou substituée comme le groupe phényle selon la définition des groupes aryle donnée ci-après, ou bien un groupe alkylidène inférieur non substitué ou mono- ou di-substitué par des groupes aryle ; et R et R₀ représentent chacun, indépendamment l'un de l'autre, l'hydrogène ou un groupe alkyle inférieur ; ou bien R et R₀ forment ensemble un groupe benzo fixé sur des atomes de carbone voisins du cycle benzénique et qui est non substitué ou substitué comme le groupe phényle selon la définition des groupes aryle donnée ci-après ; étant précisé que dans les définitions ci-dessus, lorsqu'on parle d'un groupe aryle, il s'agit dans chaque cas d'un groupe phényle non substitué ou portant un ou plusieurs substituants choisis parmi les groupes alkyle inférieurs, alcoxy inférieurs, hydroxy, alcanoyloxy inférieurs, nitro, amino, les halogènes, les groupes trifluorométhyle, carboxy, (alcoxy inférieur)carbonyle, aminocarbonyle, (alkyle inférieur)aminocarbonyle ou di-(alkyle inférieur)aminocarbonyle, cyano, alcanoyle inférieur, benzoyle, alkylsulfonyle inférieur, aminosulfonyle, (alkyle inférieur)aminosulfonyle et di-(alkyle inférieur)aminosulfonyle ; et toujours dans les définitions ci-dessus, lorsqu'on parle d'un groupe hétéroaryle, il s'agit d'un radical aromatique hétérocyclique choisi parmi les radicaux pyrrolyle, pyrazolyle, imidazolyle, triazolyle, tétrazolyle, furannyle, thiényle, isoxazolyle, oxazolyle, oxadiazolyle, isothiazolyle, thiazolyle, thiadiazolyle, pyridyle, pyridazinyle, pyrimidinyle, pyrazinyle, triazinyle, indolyle, isoindolyle, benzimidazolyle, benzotriazolyle, benzofurannyle, benzothiényle, benzoxazolyle, benzothiazolyle, benzoxadiazolyle, benzothiadiazolyle, quinoléine et isoquinoléine et qui est non substitué ou substitué comme le groupe phényle selon la définition des groupes aryle donnée ci-dessus ; et toujours dans les définitions ci-dessus, l'expression "inférieur" s'applique à un radical contenant jusqu'à 7 atomes de carbone inclus ; ou leurs sels.

2. Composés de formule I selon revendication 1, dans laquelle Tetr représente un groupe 1- ou 2-tétrazolyle ; R₁ et R₂ représentent chacun, indépendamment l'un de l'autre, l'hydrogène ; un groupe alkyle inférieur non substitué ou substitué par des groupes hydroxy, alcoxy inférieur, des halogènes, des groupes carboxy, (alcoxy inférieur)carbonyle, aminocarbonyle, (alkyle inférieur)aminocarbonyle ou di-(alkyle inférieur)aminocarbonyle ou cyano ; un groupe alcényle inférieur, aryle, hétéroaryle, aryl-alkyle inférieur, cycloalkyle en C3-C6, (cycloalkyle en C3-C6)alkyle inférieur, alkylthio inférieur, arylthio ou aryl-alkylthio inférieur ; ou bien R₁ et R₂ forment ensemble un groupe alkylène à chaîne droite en C4-C6 non substitué ou substitué par un groupe alkyle inférieur, ou bien un groupe -(CH₂)ₘ-1,2-phénylène-(CH₂)ₙ- dans lequel m et n sont égaux chacun, indépendamment l'un de l'autre, à 1 ou 2 et la partie 1,2-phénylène est non substituée ou substituée comme le groupe phényle selon la définition des groupes aryle donnée ci-après, ou bien un groupe alkylidène inférieur non substitué ou mono- ou di-substitué par des groupes aryle ; et R et R₀ représentent chacun, indépendamment l'un de l'autre, l'hydrogène ou un groupe alkyle inférieur ; ou bien R et R₀ forment ensemble un groupe benzo fixé sur des atomes de carbone voisins du cycle benzénique et qui est non substitué ou substitué comme le groupe phényle selon la définition des groupes aryle donnée ci-après ; étant précisé que dans les définitions ci-dessus, l'expression aryle désigne dans tous les cas un groupe phényle non substitué ou portant un ou deux substituants choisis parmi les groupes alkyle inférieur, alcoxy inférieur, hydroxy, alcanoyloxy inférieur, nitro, amino, les halogènes, les groupes trifluorométhyle, carboxy, (alcoxy inférieur)carbonyle, aminocarbonyle, (alkyle inférieur)aminocarbonyle, di-(alkyle inférieur)aminocarbonyle, cyano, alcanoyle inférieur, benzoyle, alkylsulfonyle inférieur, aminosulfonyle, (alkyle inférieur)aminosulfonyle ou di-(alkyle inférieur)aminosulfonyle ; et toujours dans les définitions données ci-dessus, l'expression hétéroaryle désigne un radical hétérocyclique aromatique choisi parmi les groupes thiényle, indolyle, pyridyle, furyle et benzofurannyle, et qui est non substitué ou porte un à trois substituants choisis parmi les groupes alkyle inférieur, alcoxy inférieur, cyano et les halogènes, ou bien un groupe benzotriazolyle non substitué en position 1 ou substitué par des groupes alkyle inférieur, hydroxy ou alcoxy inférieur ; ou leurs sels.

3. Composés de formule I selon revendication 1, dans laquelle Tetr représente un groupe 1- ou 2-tétrazolyle ; R₁ et R₂ représentent chacun, indépendamment l'un de l'autre, l'hydrogène ; un groupe alkyle inférieur non substitué ou substitué par un groupe (alcoxy inférieur)carbonyle ; un groupe phényle non substitué ou substitué par des groupes cyano, des halogènes, des groupes alcoxy inférieur, alkyle inférieur ou hydroxyalkyle inférieur ; un groupe thiényle, pyridyle; un groupe l-(alkyle inférieur)-lH-benzotriazolyle; un groupe phényl-alkyle inférieur non substitué dans le cycle phényle ou substitué dans le cycle phényle par un groupe cyano ; un groupe alkylthio inférieur ou phénylthio ; ou bien R₁ et R₂ forment ensemble un groupe alkylène à chaîne droite en C4-C5 ou un groupe -CH₂-1,2-phénylène-CH₂- et R et R₀ représentent l'hydrogène ; ou bien R et R₀ forment ensemble un groupe benzo fixé sur des atomes de carbone voisins du cycle benzénique ; ou leurs sels.

4. Composés de formule I selon revendication 1, dans laquelle Tetr représente un groupe 1- ou 2-tétrazolyle, R₁ représente l'hydrogène, un groupe alkyle inférieur ; un groupe phényle non substitué ou substitué par des groupes cyano, des halogènes, des groupes alcoxy inférieurs ou alkyle inférieurs ; ou bien un groupe phénylalkyle inférieur, et R, R₀ et R₂ représentent l'hydrogène ; étant précisé que dans les définitions ci-dessus, l'expression "inférieur" s'applique à des radicaux contenant jusqu'à sept atomes de carbone inclus; ou leurs sels.

5. Composés de formule I selon revendication 3, dans laquelle Tetr représente un groupe 1- ou 2-tétrazolyle ; R₁ représente l'hydrogène ; un groupe alkyle inférieur non substitué ou portant des substituants hydroxy, alcoxy inférieur, halogéno, carboxy, (alcoxy inférieur)carbonyle, aminocarbonyle, (alkyle inférieur)aminocarbonyle ou di-(alkyle inférieur)aminocarbonyle ou cyano ; un groupe alcényle inférieur, aryle, hétéroaryle, aryl-alkyle inférieur, cycloalkyle en C3-C6, (cycloalkyle en C3-C6)alkyle inférieur, alkylthio inférieur, arylthio ou aryl-alkylthio inférieur ; R₂ représente l'hydrogène ; ou bien R₁ et R₂ forment ensemble un groupe alkylène à chaîne droite en C4-C6 non substituée ou portant un substituant alkyle inférieur ; ou bien un groupe -(CH₂)ₘ-1,2-phénylène-(CH₂)ₙ- dans lequel m et n sont égaux chacun, indépendamment l'un de l'autre, à 1 ou 2 et la partie 1,2-phénylène est non substituée ou substituée comme le groupe phényle selon la définition des groupes aryle donnée ci-après ; ou bien un groupe alkylidène inférieur non substitué ou mono- ou di-substitué par des groupes aryle ; et R et R₀ représentent chacun, indépendamment l'un de l'autre, l'hydrogène ou un groupe alkyle inférieur ; ou bien R et R₀ forment ensemble un groupe benzo fixé sur des atomes de carbone voisins du cycle benzénique et qui est non substitué ou substitué comme le groupe phényle selon la définition des groupes aryle donnée ci-après ; étant précisé que dans les définitions ci-dessus, l'expression aryle désigne dans tous les cas un groupe phényle non substitué ou portant un substituant choisi parmi les groupes alkyle inférieur, alcoxy inférieur, hydroxy, alcanoyloxy inférieur, nitro, amino, les halogènes, les groupes trifluorométhyle, carboxy, (alcoxy inférieur)carbonyle, aminocarbonyle, (alkyle inférieur)aminocarbonyle ou di-(alkyle inférieur)aminocarbonyle, cyano, alcanoyle inférieur, benzoyle, alkylsulfonyle inférieur, aminosulfonyle, (alkyle inférieur)aminosulfonyle ou di-(alkyle inférieur)aminosulfonyle ; et toujours dans les définitions données ci-dessus, l'expression hétéroaryle désigne un radical hétérocyclique aromatique choisi parmi les radicaux thiényle, indolyle, pyridyle, furyle et benzofurannyle, qui est non substitué ou porte un ou deux substituants choisis parmi les groupes alkyle inférieur, alcoxy inférieur, cyano et les halogènes, ou un groupe benzotriazolyle non substitué en position 1 ou substitué par un groupe alkyle inférieur, hydroxy ou alcoxy inférieur ; ou leurs sels.

6. Composés de formule I selon revendication 3, dans laquelle Tetr représente un groupe 1- ou 2-tétrazolyle ; R₁ représente l'hydrogène ; un groupe alkyle inférieur non substitué ou substitué par un groupe (alcoxy inférieur)carbonyle ; un groupe phényle non substitué ou portant des substituants cyano, halogéno, alcoxy inférieur, alkyle inférieur ou hydroxyalkyle inférieur ; un groupe thiényle, pyridyle ; un groupe phénylalkyle inférieur non substitué ou substitué par un groupe cyano dans le cycle phényle ; un groupe alkylthio inférieur ou phénylthio ; R₂ représente l'hydrogène ; ou bien R₁ et R₂ forment ensemble un groupe alkylène à chaîne droite en C4-C5 ou un groupe -CH₂-1,2-phénylène-CH₂- ; et R et R₀ représentent l'hydrogène ou bien forment ensemble un groupe benzo fixé sur des atomes de carbone voisins du cycle benzénique ; ou leurs sels.

7. Le 4-(2-tétrazolyl)méthylbenzonitrile selon revendication 1 ou l'un de ses sels acceptables pour l'usage pharmaceutique.

8. Le 4-(1-tétrazolyl)méthylbenzonitrile selon revendication 1, ou l'un de ses sels acceptables pour l'usage pharmaceutique.

9. Le 4-[alpha-(4-cyanophényl)-(2-tétrazolyl)méthyl]benzonitrile selon revendication 1 ou l'un de ses sels acceptables pour l'usage pharmaceutique.

10. Le 4-[alpha-(4-cyanophényl)-(1-tétrazolyl)méthyl]benzonitrile selon revendication 1 ou l'un de ses sels acceptables pour l'usage pharmaceutique.

11. Compositions pharmaceutiques contenant un composé selon l'un des revendications 1 à 10 et au moins un véhicule acceptable pour l'usage pharmaceutique.

12. Un composé selon l'une des revendications 1 à 10 pour l'utilisation dans un procédé pour le traitement prophylactique ou thérapeutique de l'organisme humain ou animal.

13. Un composé selon l'une des revendications 1 à 10 pour l'utilisation dans le traitement de maladies demandant une inhibition de l'enzyme aromatase.

14. Utilisation d'un composé selon l'une des revendications 1 à 10 pour la préparation de compositions pharmaceutiques.

15. Utilisation d'un composé selon l'une des revendications 1 à 10 pour la préparation de compositions pharmaceutiques prévues pour le traitement de maladies demandant une inhibition de l'enzyme aromatase.

16. Procédé de préparation d'un composé de formule I selon revendication 1, caractérisé en ce que
(a) on fait réagir un dérivé estérifié réactif d'un composé de formule II 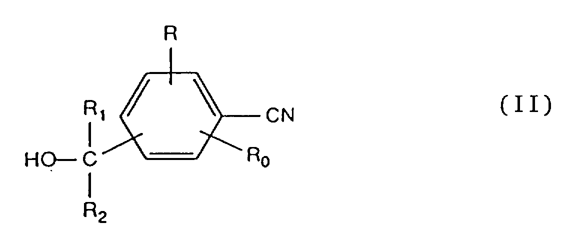 dans laquelle R, R₀, R₁ et R₂ ont les significations indiquées en référence à la formule I, avec un composé de formule III
Tetr-H (III)
dans laquelle Tetr représente un groupe 1- ou 2-tétrazolyle, ou avec un dérivé d'un tel composé protégé à l'azote, ou bien
(b) pour la préparation des composés de formule I dans laquelle Tetr représente un groupe 1-tétrazolyle, on part d'un composé de formule IV 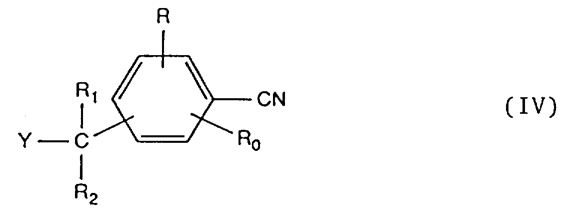 dans laquelle Y représente un groupe convertible en le groupe 1-tétrazolyle et R, R₀, R₁ et R₂ ont les significations indiquées en référence à la formule I, et on convertit Y en un groupe 1-tétrazolyle, ou bien
(c) on fait réagir un composé de formule V 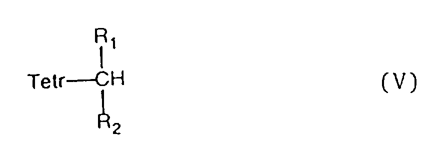 dans laquelle Tetr, R₁ et R₂ ont les significations indiquées en référence à la formule I, en milieu basique, avec un composé de formule VI 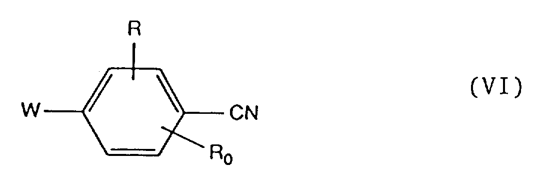 dans laquelle W représente un groupe éliminable et R et R₀ ont les significations indiquées en référence à la formule I, ou bien
(d) on part d'un composé de formule VII 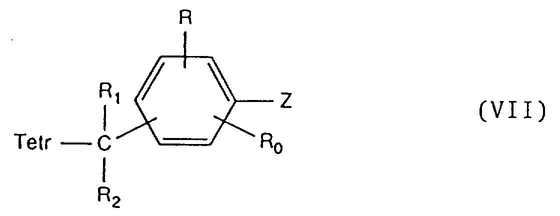 dans laquelle Z représente un groupe convertible en groupe cyano et Tetr, R, R₀, R₁ et R₂ ont les significations indiquées en référence à la formule I, et on convertit Z en un groupe cyano ; et/ou, si on le désire, on convertit un composé de formule I ainsi obtenu en un autre composé de formule I, et/ou, si on le désire, on convertit un sel ainsi obtenu en le composé libre ou en un autre sel, et/ou, si on le désire, on convertit un composé de formule I obtenu à l'état libre en un sel et/ou on sépare un mélange de composés isomères de formule I en les isomères individuels.

## Revendications (Revendications pour l'(les) Etat(s) contractant(s) suivant(s): ES, GR)

1. Procédé de préparation des composés de formule I 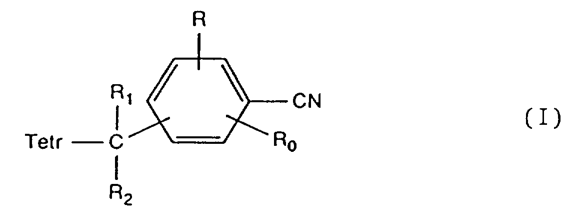 dans laquelle Tetr représente un groupe 1- ou 2-tétrazolyle qui est non substitué ou substitué en position 5 par un groupe alkyle inférieur, phényl-alkyle inférieur ou alcanoyle inférieur ; R₁ et R₂ représentent chacun, indépendamment l'un de l'autre, l'hydrogène ; un groupe alkyle inférieur non substitué ou substitué par des groupes hydroxy, alcoxy inférieur, des halogènes, des groupes carboxy, (alcoxy inférieur)carbonyle, aminocarbonyle, (alkyle inférieur)aminocarbonyle ou di-(alkyle inférieur)aminocarbonyle ou cyano ; un groupe alcényle inférieur, aryle, hétéroaryle, aryl-alkyle inférieur, cycloalkyle en C3-C6, (cycloalkyle en C3-C6)alkyle inférieur, alkylthio inférieur, arylthio ou aryl-(alkyle inférieur)thio ; ou bien R₁ et R₂ forment ensemble un groupe alkylène à chaîne droite en C4-C6, non substitué ou substitué par un groupe alkyle inférieur, ou un groupe -(C_{H}2)ₘ-1,2-phénylène-(CH₂)ₙ- dans lequel m et n sont égaux chacun, indépendamment l'un de l'autre, à 1 ou 2 et la partie 1,2-phénylène est non substituée ou substituée comme le groupe phényle selon la définition des groupes aryle donnée ci-après, ou bien un groupe alkylidène inférieur non substitué ou mono- ou di-substitué par des groupes aryle ; et R et R₀ représentent chacun, indépendamment l'un de l'autre, l'hydrogène ou un groupe alkyle inférieur ; ou bien R et R₀ forment ensemble un groupe benzo fixé sur des atomes de carbone voisins du cycle benzénique et qui est non substitué ou substitué comme le groupe phényle selon la définition des groupes aryle donnée ci-après ; étant précisé que dans les définitions ci-dessus, lorsqu'on parle d'un groupe aryle, il s'agit dans chaque cas d'un groupe phényle non substitué ou portant un ou plusieurs substituants choisis parmi les groupes alkyle inférieurs, alcoxy inférieurs, hydroxy, alcanoyloxy inférieurs, nitro, amino, les halogènes, les groupes trifluorométhyle, carboxy, (alcoxy inférieur)carbonyle, aminocarbonyle, (alkyle inférieur)aminocarbonyle ou di-(alkyle inférieur)aminocarbonyle, cyano, alcanoyle inférieur, benzoyle, alkylsulfonyle inférieur, aminosulfonyle, (alkyle inférieur)aminosulfonyle et di-(alkyle inférieur)aminosulfonyle ; et toujours dans les définitions ci-dessus, lorsqu'on parle d'un groupe hétéroaryle, il s'agit d'un radical aromatique hétérocyclique choisi parmi les radicaux pyrrolyle, pyrazolyle, imidazolyle, triazolyle, tétrazolyle, furannyle, thiényle, isoxazolyle, oxazolyle, oxadiazolyle, isothiazolyle, thiazolyle, thiadiazolyle, pyridyle, pyridazinyle, pyrimidinyle, pyrazinyle, triazinyle, indolyle, isoindolyle, benzimidazolyle, benzotriazolyle, benzofurannyle, benzothiényle, benzoxazolyle, benzothiazolyle, benzoxadiazolyle, benzothiadiazolyle, quinoléine et isoquinoléine et qui est non substitué ou substitué comme le groupe phényle selon la définition des groupes aryle donnée ci-dessus ; et toujours dans les définitions ci-dessus, l'expression "inférieur" s'applique à un radical contenant jusqu'à 7 atomes de carbone inclus ; ou de leurs sels, caractérisé en ce que
(a) on fait réagir un dérivé estérifié réactif d'un composé de formule II 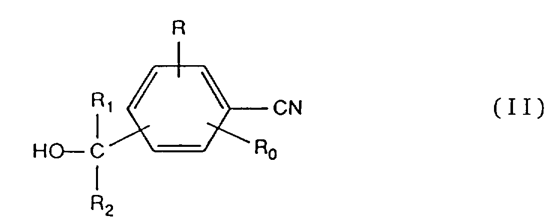 dans laquelle R, R₀, R₁ et R₂ ont les significations indiquées en référence à la formule I, avec un composé de formule III
Tetr-H (III)
dans laquelle Tetr représente un groupe 1- ou 2-tétrazolyle, ou avec un dérivé d'un tel composé protégé à l'azote, ou bien
(b) pour la préparation des composés de formule I dans laquelle Tetr représente un groupe 1-tétrazolyle, on part d'un composé de formule IV 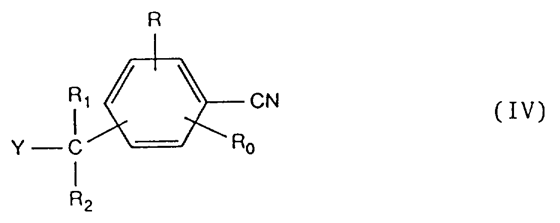 dans laquelle Y représente un groupe convertible en le groupe 1-tétrazolyle et R, R₀, R₁ et R₂ ont les significations indiquées en référence à la formule I, et on convertit Y en un groupe 1-tétrazolyle, ou bien
(c) on fait réagir un composé de formule V 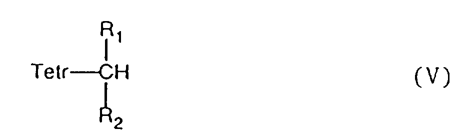 dans laquelle Tetr, R₁ et R₂ ont les significations indiquées en référence à la formule I, en milieu basique, avec un composé de formule VI 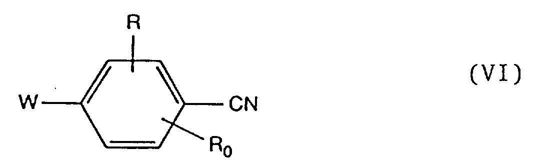 dans laquelle W représente un groupe éliminable et R et R₀ ont les significations indiquées en référence à la formule I, ou bien
(d) on part d'un composé de formule VII 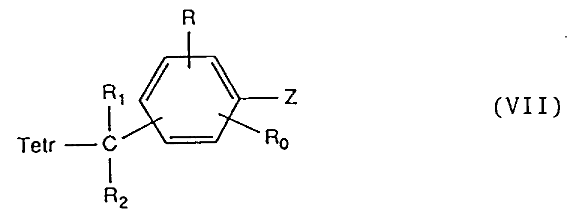 dans laquelle Z représente un groupe convertible en groupe cyano et Tetr, R, R₀, R₁ et R₂ ont les significations indiquées en référence à la formule I, et on convertit Z en un groupe cyano ; et/ou, si on le désire, on convertit un composé de formule I ainsi obtenu en un autre composé de formule I, et/ou, si on le désire, on convertit un sel ainsi obtenu en le composé libre ou en un autre sel, et/ou, si on le désire, on convertit un composé de formule I obtenu à l'état libre en un sel et/ou on sépare un mélange de composés isomères de formule I en les isomères individuels.

2. Procédé selon revendication 1 pour la préparation des composés de formule I dans laquelle Tetr représente un groupe 1- ou 2-tétrazolyle ; R₁ et R₂ représentent chacun, indépendamment l'un de l'autre, l'hydrogène ; un groupe alkyle inférieur non substitué ou substitué par des groupes hydroxy, alcoxy inférieur, des halogènes, des groupes carboxy, (alcoxy inférieur)carbonyle, aminocarbonyle, (alkyle inférieur)aminocarbonyle ou di-(alkyle inférieur)aminocarbonyle ou cyano; un groupe alcényle inférieur, aryle, hétéroaryle, aryl-alkyle inférieur, cycloalkyle en C3-C6, (cycloalkyle en C3-C6)alkyle inférieur, alkylthio inférieur, arylthio ou aryl-alkylthio inférieur ; ou bien R₁ et R₂ forment ensemble un groupe alkylène à chaîne droite en C4-C6 non substitué ou substitué par un groupe alkyle inférieur, ou bien un groupe -(CH₂)ₘ-1,2-phénylène-(CH₂)ₙ- dans lequel m et n sont égaux chacun, indépendamment l'un de l'autre, à 1 ou 2 et la partie 1,2-phénylène est non substituée ou substituée comme le groupe phényle selon la définition des groupes aryle donnée ci-après, ou bien un groupe alkylidène inférieur non substitué ou mono- ou di-substitué par des groupes aryle ; et R et R₀ représentent chacun, indépendamment l'un de l'autre, l'hydrogène ou un groupe alkyle inférieur ; ou bien R et R₀ forment ensemble un groupe benzo fixé sur des atomes de carbone voisins du cycle benzénique et qui est non substitué ou substitué comme le groupe phényle selon la définition des groupes aryle donnée ci-après ; étant précisé que dans les définitions ci-dessus, l'expression aryle désigne dans tous les cas un groupe phényle non substitué ou portant un ou deux substituants choisis parmi les groupes alkyle inférieur, alcoxy inférieur, hydroxy, alcanoyloxy inférieur, nitro, amino, les halogènes, les groupes trifluorométhyle, carboxy, (alcoxy inférieur)carbonyle, aminocarbonyle, (alkyle inférieur)aminocarbonyle, di-(alkyle inférieur)aminocarbonyle, cyano, alcanoyle inférieur, benzoyle, alkylsulfonyle inférieur, aminosulfonyle, (alkyle inférieur)aminosulfonyle ou di-(alkyle inférieur)aminosulfonyle ; et toujours dans les définitions données ci-dessus, l'expression hétéroaryle désigne un radical hétérocyclique aromatique choisi parmi les groupes thiényle, indolyle, pyridyle, furyle et benzofurannyle, et qui est non substitué ou porte un à trois substituants choisis parmi les groupes alkyle inférieur, alcoxy inférieur, cyano et les halogènes, ou bien un groupe benzotriazolyle non substitué en position 1 ou substitué par des groupes alkyle inférieur, hydroxy ou alcoxy inférieur ; ou leurs sels, caractérisé en ce que l'on utilise les produits de départ portant les substituants appropriés.

3. Procédé selon la revendication 1 pour la préparation des composés de formule I dans laquelle Tetr représente un groupe 1- ou 2-tétrazolyle ; R₁ et R₂ représentent chacun, indépendamment l'un de l'autre, l'hydrogène ; un groupe alkyle inférieur non substitué ou substitué par un groupe (alcoxy inférieur)carbonyle ; un groupe phényle non substitué ou substitué par des groupes cyano, des halogènes, des groupes alcoxy inférieur, alkyle inférieur ou hydroxyalkyle inférieur ; un groupe thiényle, pyridyle ; un groupe 1-(alkyle inférieur)-1H-benzotriazolyle ; un groupe phényl-alkyle inférieur non substitué dans le cycle phényle ou substitué dans le cycle phényle par un groupe cyano ; un groupe alkylthio inférieur ou phénylthio ; ou bien R₁ et R₂ forment ensemble un groupe alkylène à chaîne droite en C4-C5 ou un groupe -CH₂-1,2-phénylène-CH₂- ; et R et R₀ représentent l'hydrogène ; ou bien R et R₀ forment ensemble un groupe benzo fixé sur des atomes de carbone voisins du cycle benzénique ; ou leurs sels, caractérisé en ce que l'on utilise des produits de départ portant les substituants correspondants.

4. Procédé selon revendication 1 pour la préparation des composés de formule I dans laquelle Tetr représente un groupe 1- ou 2-tétrazolyle, R₁ représente l'hydrogène, un groupe alkyle inférieur ; un groupe phényle non substitué ou substitué par des groupes cyano, des halogènes, des groupes alcoxy inférieurs ou alkyle inférieurs ; ou bien un groupe phénylalkyle inférieur, et R, R₀ et R₂ représentent l'hydrogène ; étant précisé que dans les définitions ci-dessus, l'expression "inférieur" s'applique à des radicaux contenant jusqu'à sept atomes de carbones inclus ; ou leurs sels, caractérisé en ce que l'on utilise des produits de départ portant les substituants correspondants.

5. Procédé selon revendication 1 pour la préparation des composés de formule I dans laquelle Tetr représente un groupe 1- ou 2-tétrazolyle ; R₁ représente l'hydrogène ; un groupe alkyle inférieur non substitué ou portant des substituants hydroxy, alcoxy inférieur, halogéno, carboxy, (alcoxy inférieur)carbonyle, aminocarbonyle, (alkyle inférieur)aminocarbonyle ou di-(alkyle inférieur)aminocarbonyle ou cyano ; un groupe alcényle inférieur, aryle, hétéroaryle, aryl-alkyle inférieur, cycloalkyle en C3-C6, (cycloalkyle en C3-C6)alkyle inférieur, alkylthio inférieur, arylthio ou aryl-alkylthio inférieur ; R₂ représente l'hydrogène ; ou bien R₁ et R₂ forment ensemble un groupe alkylène à chaîne droite en C4-C6 non substituée ou portant un substituant alkyle inférieur ; ou bien un groupe -(CH₂)ₘ-1,2-phénylène-(CH₂)ₙ- dans lequel m et n sont égaux chacun, indépendamment l'un de l'autre, à 1 ou 2 et la partie 1,2-phénylène est non substituée ou substituée comme le groupe phényle selon la définition des groupes aryle donnée ci-après ; ou bien un groupe alkylidène inférieur non substitué ou mono- ou di-substitué par des groupes aryle ; et R et R₀ représentent chacun, indépendamment l'un de l'autre, l'hydrogène ou un groupe alkyle inférieur ; ou bien R et R₀ forment ensemble un groupe benzo fixé sur des atomes de carbone voisins du cycle benzénique et qui est non substitué ou substitué comme le groupe phényle selon la définition des groupes aryle donnée ci-après ; étant précisé que dans les définitions ci-dessus, l'expression aryle désigne dans tous les cas un groupe phényle non substitué ou portant un substituant choisi parmi les groupes alkyle inférieur, alcoxy inférieur, hydroxy, alcanoyloxy inférieur, nitro, amino, les halogènes, les groupes trifluorométhyle, carboxy, (alcoxy inférieur)carbonyle, aminocarbonyle, (alkyle inférieur)aminocarbonyle ou di-(alkyle inférieur)aminocarbonyle, cyano, alcanoyle inférieur, benzoyle, alkylsulfonyle inférieur, aminosulfonyle, (alkyle inférieur)aminosulfonyle ou di-(alkyle inférieur)aminosulfonyle ; et toujours dans les définitions données ci-dessus, l'expression hétéroaryle désigne un radical hétérocyclique aromatique choisi parmi les radicaux thiényle, indolyle, pyridyle, furyle et benzofurannyle, qui est non substitué ou porte un ou deux substituants choisis parmi les groupes alkyle inférieur, alcoxy inférieur, cyano et les halogènes, ou un groupe benzotriazolyle non substitué en position 1 ou substitué par un groupe alkyle inférieur, hydroxy ou alcoxy inférieur ; ou leurs sels, caractérisé en ce que l'on utilise des produits de départ portant les substituants appropriés.

6. Procédé selon revendication 4 pour la préparation des composés de formule I dans laquelle Tetr représente un groupe 1- ou 2-tétrazolyle ; R₁ représente l'hydrogène ; un groupe alkyle inférieur non substitué ou substitué par un groupe (alcoxy inférieur)carbonyle; un groupe phényle non substitué ou portant des substituants cyano, halogéno, alcoxy inférieur, alkyle inférieur ou hydroxyalkyle inférieur ; un groupe thiényle, pyridyle ; un groupe phénylalkyle inférieur non substitué ou substitué par un groupe cyano dans le cycle phényle ; un groupe alkylthio inférieur ou phénylthio ; R₂ représente l'hydrogène ; ou bien R₁ et R₂ forment ensemble un groupe alkylène à chaîne droite en C4-C5 ou un groupe -CH₂-1,2-phénylène-CH₂- ; et R et R₀ représentent l'hydrogène ou bien forment ensemble un groupe benzo fixé sur des atomes de carbone voisins du cycle benzénique ; ou leurs sels, caractérisé en ce que l'on utilise des produits de départ portant les substituants appropriés.

7. Procédé selon revendication 1 pour la préparation du composé de formule I consistant en le 4-(2-tétrazolyl)méthylbenzonitrile, ou de l'un de ses sels acceptables pour l'usage pharmaceutique, caractérisé en ce que l'on utilise des produits de départ portant les substituants appropriés.

8. Procédé selon revendication 1 pour la préparation d'un composé de formule I consistant en le 4-(1-tétrazolyl)méthylbenzonitrile ou de l'un de ses sels acceptables pour l'usage pharmaceutique, caractérisé en ce que l'on utilise des produits de départ portant les substituants appropriés.

9. Procédé selon revendication 1 pour la préparation d'un composé de formule I consistant en le 4-[alpha-(4-cyanophényl)-(2-tétrazolyl)méthyl]benzonitrile ou l'un de ses sels acceptables pour l'usage pharmaceutique, caractérisé en ce que l'on utilise des produits de départ portant les substituants apprppriés.

10. Procédé selon revendication 1 pour la préparation d'un composé de formule I consistant en le 4-[alpha-(4-cyanophényl)-(1-tétrazolyl)méthyl]benzonitrile selon revendication 1 ou l'un de ses sels acceptables pour l'usage pharmaceutique, caractérisé en ce que l'on utilise des produits de départ portant les substituants appropriés.
